# EUROPEAN PATENT APPLICATION

(11) **EP 1 502 955 A1**
(43) Date of publication of application: **02.02.2005**
(21) Application number: 03017415.5
(22) Date of filing: 01.08.2003
(51) Int. Cl.: C12N 15/82, A01H 5/00

(54) **Methods for the production of stably transformed, fertile gramineae employing agrobacterium-mediated transformation of isolated gramineae zygotes**

(71) Applicant: Institut für Pflanzengenetik und Kulturpflanzenforschung, 06466 Gatersleben (DE)
(72) Inventor: Kumlehn, Jochen, 14469 Potsdam (DE)
(74) Representative: Baumbach, Friedrich, Dr.

(57) **Abstract**

The present invention relates to methods for the incorporation of DNA into the genome of a Gramineae plant, preferably a wheat plant, comprising the steps of:
(a) isolating a zygote from a Gramineae plant to be transformed in a way that said isolated zygote becomes substantially free from its naturally surrounding tissue,
(b) introducing a DNA composition comprising a genetic component into the genome of said Gramineae plant, wherein said introduction is mediated by Agrobacterium transformation into said isolated zygote;
(c) regenerating Gramineae plants from said zygotes which have received said genetic component; and
(d) identifying a fertile, transgenic Gramineae plant whose genome has been altered through the stable introduction of said genetic component.

SEQUENCE LISTING

<110> Institut für Pflanzengenetik und Kulturpflanzenforschung

<120> METHODS FOR THE PRODUCTION OF STABLY TRANSFORMED, FERTILE
GRAMINEAE EMPLOYING AGROBACTERIUM-MEDIATED TRANSFORMATION OF ISOLATED
GRAMINEAE ZYGOTES

<130> AE20030363

<160> 3

<170> PatentIn version 3.1

<210> 1
<211> 12739
<212> DNA
<213> synthetic: expression vector - binary vector pUGAB7

<400> 1
tctagaacat ggtggagcac gacactctcg tctactccaa gaatatcaaa gatacagtct 60
cagaagacca gagggctatt gagacttttc aacaaagggt aatatcggga aacctcctcg 120
gattccattg cccagctatc tgtcacttca tcgaaaggac agtagaaaag gaagatggct 180
tctacaaatg ccatcattgc gataaaggaa aggctatcgt tcaagatgcc tctaccgaca 240
gtggtcccaa agatggaccc ccacccacga ggaacatcgt ggaaaaagaa gacgttccaa 300
ccacgtcttc aaagcaagtg gattgatgtg atatctccac tgacgtaagg gatgacgcac 360
aatcccacta tccttcgcaa gacccttcct ctatataagg aagttcattt catttggaga 420
ggacctcgag tggccaccat gggcccagaa cgacgcccgg ccgacatccg ccgtgccacc 480
gaggcggaca tgccggcggt ctgcaccatc gtcaaccact acatcgagac aagcacggtc 540
aacttccgta ccgagccgca ggaaccgcag gagtggacgg acgacctcgt ccgtctgcgg 600
gagcgctatc cctggctcgt cgccgaggtg gacggcgagg tcgccggcat cgcctacgcg 660
ggcccctgga aggcacgcaa cgcctacgac tggacggccg agtcgaccgt gtacgtctcc 720
ccccgccacc agcggacggg actgggctcc acgctctaca cccacctgct gaagtccctg 780
gaggcacagg gcttcaagag cgtggtcgct gtcatcgggc tgcccaacga cccgagcgtg 840
cgcatgcacg aggcgctcgg atatgccccc cgcggcatgc tgcgggcggc cggcttcaag 900
cacgggaact ggcatgacgt gggtttctgg cagctggact tcagcctgcc ggtaccgccc 960
cgtccggtcc tgcccgtcac cgagatctga gatcacgcgt tctagtccgc aaaaatcacc 1020
agtctctctc tacaaatcta tctctctcta tttttctcca gaataatgtg tgagtagttc 1080
ccagataagg gaattagggt tcttataggg tttcgctcat gtgttgagca tataagaaac 1140
ccttagtatg tatttgtatt tgtaaaatac ttctatcaat aaaatttcta attcctaaaa 1200
ccaaaatcca gtgacctgca ggcatgcaag ctgatccact agaggccatg gcggccgcgt 1260
cgagcgatct agtaacatag atgacaccgc gcgcgataat ttatcctagt ttgcgcgcta 1320
tattttgttt tctatcgcgt attaaatgta taattgcggg actctaatca taaaaaccca 1380
tctcataaat aacgtcatgc attacatgtt aattattaca tgcttaacgt aattcaacag 1440
aaattatatg ataatcatcg caagaccggc aacaggattc aatcttaaga aactttattg 1500
ccaaatgttt gaacgatcgg ggaaattcga gctcggtagc aattcccgag gctgtagccg 1560
acgatggtgc gccaggagag ttgttgattc attgtttgcc tccctgctgc ggtttttcac 1620
cgaagttcat gccagtccag cgtttttgca gcagaaaagc cgccgacttc ggtttgcggt 1680
cgcgagtgaa gatccctttc ttgttaccgc caacgcgcaa tatgccttgc gaggtcgcaa 1740

aatcggcgaa attccatacc tgttcaccga cgacggcgct gacgcgatca aagacgcggt 1800
gatacatatc cagccatgca cactgatact cttcactcca catgtcggtg tacattgagt 1860
gcagcccggc taacgtatcc acgccgtatt cggtgatgat aatcggctga tgcagtttct 1920
cctgccaggc cagaagttct ttttccagta ccttctctgc cgtttccaaa tcgccgcttt 1980
ggacatacca tccgtaataa cggttcaggc acagcacatc aaagagatcg ctgatggtat 2040
cggtgtgagc gtcgcagaac attacattga cgcaggtgat cggacgcgtc gggtcgagtt 2100
tacgcgttgc ttccgccagt ggcgcgaaat attcccgtgc accttgcgga cgggtatccg 2160
gttcgttggc aatactccac atcaccacgc ttgggtggtt tttgtcacgc gctatcagct 2220
ctttaatcgc ctgtaagtgc gcttgctgag tttccccgtt gactgcctct tcgctgtaca 2280
gttctttcgg cttgttgccc gcttcgaaac caatgcctaa agagaggtta aagccgacag 2340
cagcagtttc atcaatcacc acgatgccat gttcatctgc ccagtcgagc atctcttcag 2400
cgtaagggta atgcgaggta cggtaggagt tggccccaat ccagtccatt aatgcgtggt 2460
cgtgcaccat cagcacgtta tcgaatcctt tgccacgcaa gtccgcatct tcatgacgac 2520
caaagccagt aaagtagaac ggtttgtggt taatcaggaa ctgttggccc ttcactgcca 2580
ctgaccggat gccgacgcga agcgggtaga tatcacactc tgtctggctt ttggctgtga 2640
cgcacagttc atagagataa ccttcacccg gttgccagag gtgcggattc accacttgca 2700
aagtcccgct agtgccttgt ccagttgcaa ccacctgttg atccgcatca cgcagttcaa 2760
cgctgacatc accattggcc accacctgcc agtcaacaga cgcgtggtta cagtcttgcg 2820
cgacatgcgt caccacggtg atatcgtcca cccaggtgtt cggcgtggtg tagagcatta 2880
cgctgcgatg gattccggca tagttaaaga aatcatggaa gtaagactgc tttttcttgc 2940
cgttttcgtc ggtaatcacc attcccggcg ggatagtctg ccagttcagt tcgttgttca 3000
cacaaacggt gataccagct gcacatcaac aaattttggt catatattag aaaagttata 3060
aattaaaata tacacactta taaactacag aaaagcaatt gctatatact acattctttt 3120
attttgaaaa aaatatttga aatattatat tactactaat taatgataat tattatatat 3180
atatcaaagg tagaagcaga aacttacgta cacttttccc ggcaataaca tacggcgtga 3240
catcggcttc aaatggcgta tagccgccct gatgctccat cacttcctga ttattgaccc 3300
acactttgcc gtaatgagtg accgcatcga aacgcagcac gatacgctgg cctgcccaac 3360
ctttcggtat aaagacttcg cgctgatacc agacgttgcc cgcataatta cgaatatctg 3420
catcggcgaa ctgatcgtta aaactgcctg gcacagcaat tgcccggctt tcttgtaacg 3480
cgctttccca ccaacgctga ccaattccac agttttcgcg atccagactg aatgcccaca 3540
ggccgtcgag ttttttgatt tcacgggttg gggtttctac aggacgtaac ataagggact 3600
gacgtcgaat ccactagtac ctgcagaagt aacaccaaac aacagggtga gcatcgacaa 3660
aagaaacagt accaagcaaa taaatagcgt atgaaggcag ggctaaaaaa atccacatat 3720
agctgctgca tatgccatca tccaagtata tcaagatcaa aataattata aaacatactt 3780
gtttattata atagataggt actcaaggtt agagcatatg aatagatgct gcatatgcca 3840
tcatgtatat gcatcagtaa aacccacatc aacatgtata cctatcctag atcgatcccg 3900
tctgcggaac ggctagagcc atcccaggat tccccaaaga gaaacactgg caagttagca 3960
atcagaacgt gtctgacgta caggtcgcat ccgtgtacga acgctagcag cacggatcta 4020
acacaaacac ggatctaaca caaacatgaa cagaagtaga actaccgggc cctaaccatg 4080
gaccggaacg ccgatctaga gaaggtagag aggggggggg ggggaggacg agcggcgtac 4140
cttgaagcgg aggtgccgac gggtggattt gggggagatc tggttgtgtg tgtgtgcgct 4200
ccgaacaaca cgaggttggg gaaagagggt gtggaggggg tgtctattta ttacggcggg 4260
cgaggaaggg aaagcgaagg agcggtggga aaggaatccc ccgtagctgc cggtgccgtg 4320
agaggaggag gaggccgcct gccgtgccgg ctcacgtctg ccgctccgcc acgcaatttc 4380
tggatgccga cagcggagca agtccaacgg tggagcggaa ctctcgagag gggtccagag 4440
gcagcgacag agatgccgtg ccgtctgctt cgcttggccc gacgcgacgc tgctggttcg 4500
ctggttggtg tccgttagac tcgtcgatcg acggcgttta acaggctggc attatctact 4560
cgaaacaaga aaaatgtttc cttagttttt ttaatttctt aaagggtatt tgtttaattt 4620
ttagtcactt tattttattc tattttatat ctaaattatt aaataaaaaa actaaaatag 4680

agttttagtt ttcttaattt agaggctaaa atagaataaa atagatgtac taaaaaaatt 4740
agtctataaa aaccattaac cctaaaccct aaatggatgt actaataaaa tggatgaagt 4800
attatatagg tgaagctatt tgcaaaaaaa aaggagaaca catgcacact aaaaagataa 4860
aactgtagag tcctgttgtc aaaatactca attgtccttt agaccatgtc taactgttca 4920
tttatatgat tctctaaaac actgatatta ttgtagtact atagattata ttattcgtag 4980
agtaaagttt aaatatatgt ataaagatag ataaactgca cttcaaacaa gtgtgacaaa 5040
aaaaatatgt ggtaattttt tataacttag acatgcaatg ctcattatct ctagagaggg 5100
gcacgaccgg gtcacgctgc actgcagcct agtaaggcct taagggccag atcttgggcc 5160
cggtacccga tcagattgtc gtttcccgcc ttcggtttaa actatcagtg tttgacagga 5220
tatattggcg ggtaaaccta agagaaaaga gcgtttatta gaataatcgg atatttaaaa 5280
gggcgtgaaa aggtttatcc gttcgtccat ttgtatgtgc atgccaacca cagggttccc 5340
ctcgggagtg cttggcattc cgtgcgataa tgacttctgt tcaaccaccc aaacgtcgga 5400
aagcctgacg acggagcagc attccaaaaa gatcccttgg ctcgtctggg tcggctagaa 5460
ggtcgagtgg gctgctgtgg cttgatccct caacgcggtc gcggacgtag cgcagcgccg 5520
aaaaatcctc gatcgcaaat ccgacgctgt cgaaaagcgt gatctgcttg tcgctctttc 5580
ggccgacgtc ctggccagtc atcacgcgcc aaagttccgt cacaggatga tctggcgcga 5640
gttgctggat ctcgccttca atccgggtct gtggcgggaa ctccacgaaa atatccgaac 5700
gcagcaagat atcgcggtgc atctcggtct tgcctgggca gtcgccgccg acgccgttga 5760
tgtggacgcc gaaaaggatc taggtgaaga tcctttttga taatctcatg accaaaatcc 5820
cttaacgtga gttttcgttc cactgagcgt cagaccccgt agaaaagatc aaaggatctt 5880
cttgagatcc tttttttctg cgcgtaatct gctgcttgca aacaaaaaaa ccaccgctac 5940
cagcggtggt ttgtttgccg gatcaagagc taccaactct ttttccgaag gtaactggct 6000
tcagcagagc gcagatacca aatactgttc ttctagtgta gccgtagtta ggccaccact 6060
tcaagaactc tgtagcaccg cctacatacc tcgctctgct aatcctgtta ccagtggctg 6120
ctgccagtgg cgataagtcg tgtcttaccg ggttggactc aagacgatag ttaccggata 6180
aggcgcagcg gtcgggctga acggggggtt cgtgcacaca gcccagcttg gagcgaacga 6240
cctacaccga actgagatac ctacagcgtg agctatgaga aagcgccacg cttcccgaag 6300
ggagaaaggc ggacaggtat ccggtaagcg gcagggtcgg aacaggagag cgcacgaggg 6360
agcttccagg gggaaacgcc tggtatcttt atagtcctgt cgggtttcgc cacctctgac 6420
ttgagcgtcg atttttgtga tgctcgtcag gggggcggag cctatggaaa aacgccagca 6480
acgcggcctt tttacggttc ctggcctttt gctggccttt tgctcacatg ttctttcctg 6540
cgttatcccc tgattctgtg gataaccgat taccgccttt gagtgagctg ataccgctcg 6600
ccgcagccga acgaccgagc gcagcgagtc agtgagcgag gaagcggaag agcgcctgat 6660
gcggtatttt ctccttacgc atctgtgcgg tatttcacac cgcatatggt gcactctcag 6720
tacaatctgc tctgatgccg catagttaag ccagtataca ctccgctatc gctacgtgac 6780
tgggtcatgg ctgcgccccg acacccgcca acacccgctg acgcgccctg acgggcttgt 6840
ctgctcccgg catccgctta cagacaagct gtgaccgtct ccgggagctg catgtgtcag 6900
aggttttcac cgtcatcacc gaaacgcgcg aggcaggggt acgtcgaggt cgatccaacc 6960
cctccgctgc tatagtgcag tcggcttctg acgttcagtg cagccgtctt ctgaaaacga 7020
catgtcgcac aagtcctaag ttacgcgaca ggctgccgcc ctgccctttt cctggcgttt 7080
tcttgtcgcg tgttttagtc gcataaagta gaatacttgc gactagaacc ggagacatta 7140
cgccatgaac aagagcgccg ccgctggcct gctgggctat gcccgcgtca gcaccgacga 7200
ccaggacttg accaaccaac gggccgaact gcacgcggcc ggctgcacca agctgttttc 7260
cgagaagatc accggcacca ggcgcgaccg cccggagctg gccaggatgc ttgaccacct 7320
acgccctggc gacgttgtga cagtgaccag gctagaccgc ctggcccgca gcacccgcga 7380
cctactggac attgccgagc gcatccagga ggccggcgcg ggcctgcgta gcctggcaga 7440
gccgtgggcc gacaccacca cgccggccgg ccgcatggtg ttgaccgtgt tcgccggcat 7500
tgccgagttc gagcgttccc taatcatcga ccgcacccgg agcgggcgcg aggccgccaa 7560
ggcgcgaggc gtgaagtttg gcccccgccc taccctcacc ccggcacaga tcgcgcacgc 7620

ccgcgagctg atcgaccagg aaggccgcac cgtgaaagag gcggctgcac tgcttggcgt 7680
gcatcgctcg accctgtacc gcgcacttga gcgcagcgag gaagtgacgc ccaccgaggc 7740
caggcggcgc ggtgccttcc gtgaggacgc attgaccgag gccgacgccc tggcggccgc 7800
cgagaatgaa cgccaagagg aacaagcatg aaaccgcacc aggacggcca ggacgaaccg 7860
tttttcatta ccgaagagat cgaggcggag atgatcgcgg ccgggtacgt gttcgagccg 7920
cccgcgcacg tctcaaccgt gcggctgcat gaaatcctgg ccggtttgtc tgatgccaag 7980
ctcgcggcct ggccggcgag cttggccgct gaagaaaccg agcgccgccg tctaaaaagg 8040
tgatgtgtat ttgagtaaaa cagcttgcgt catgcggtcg ctgcgtatat gatgcgatga 8100
gtaaataaac aaatacgcaa ggggaacgca tgaaggttat cgctgtactt aaccagaaag 8160
gcgggtcagg caagacgacc atcgcaaccc atctagcccg cgccctgcaa ctcgccgggg 8220
ccgatgttct gttagtcgat tccgatcccc agggcagtgc ccgcgattgg gcggccgtgc 8280
gggaagatca accgctaacc gttgtcggca tcgaccgccc gacgattgac cgcgacgtga 8340
aggccatcgg ccggcgcgac ttcgtagtga tcgacggagc gccccaggcg gcggacttgg 8400
ctgtgtccgc gatcaaggca gccgacttcg tgctgattcc ggtgcagcca agcccttacg 8460
acatatgggc caccgccgac ctggtggagc tggttaagca gcgcattgag gtcacggatg 8520
gaaggctaca agcggccttt gtcgtgtcgc gggcgatcaa aggcacgcgc atcggcggtg 8580
aggttgccga ggcgctggcc gggtacgagc tgcccattct tgagtcccgt atcacgcagc 8640
gcgtgagcta cccaggcact gccgccgccg gcacaaccgt tcttgaatca gaacccgagg 8700
gcgacgctgc ccgcgaggtc caggcgctgg ccgctgaaat taaatcaaaa ctcatttgag 8760
ttaatgaggt aaagagaaaa tgagcaaaag cacaaacacg ctaagtgccg gccgtccgag 8820
cgcacgcagc agcaaggctg caacgttggc cagcctggca gacacgccag ccatgaagcg 8880
ggtcaacttt cagttgccgg cggaggatca caccaagctg aagatgtacg cggtacgcca 8940
aggcaagacc attaccgagc tgctatctga atacatcgcg cagctaccag agtaaatgag 9000
caaatgaata aatgagtaga tgaattttag cggctaaagg aggcggcatg gaaaatcaag 9060
aacaaccagg caccgacgcc gtggaatgcc ccatgtgtgg aggaacgggc ggttggccag 9120
gcgtaagcgg ctgggttgtc tgccggccct gcaatggcac tggaaccccc aagcccgagg 9180
aatcggcgtg agcggtcgca aaccatccgg cccggtacaa atcggcgcgg cgctgggtga 9240
tgacctggtg gagaagttga aggcggcgca ggccgcccag cggcaacgca tcgaggcaga 9300
agcacgcccc ggtgaatcgt ggcaagcggc cgctgatcga atccgcaaag aatcccggca 9360
accgccggca gccggtgcgc cgtcgattag gaagccgccc aagggcgacg agcaaccaga 9420
ttttttcgtt ccgatgctct atgacgtggg cacccgcgat agtcgcagca tcatggacgt 9480
ggccgttttc cgtctgtcga agcgtgaccg acgagctggc gaggtgatcc gctacgagct 9540
tccagacggg cacgtagagg tttccgcagg gccggccggc atggcgagtg tgtgggatta 9600
cgacctggta ctgatggcgg tttcccatct aaccgaatcc atgaaccgat accgggaagg 9660
gaagggagac aagcccggcc gcgtgttccg tccacacgtt gcggacgtac tcaagttctg 9720
ccggcgagcc gatggcggaa agcagaaaga cgacctggta gaaacctgca ttcggttaaa 9780
caccacgcac gttgccatgc agcgtacgaa gaaggccaag aacggccgcc tggtgacggt 9840
atccgagggt gaagccttga ttagccgcta caagatcgta aagagcgaaa ccgggcggcc 9900
ggagtacatc gagatcgagc tagctgattg gatgtaccgc gagatcacag aaggcaagaa 9960
cccggacgtg ctgacggttc accccgatta ctttttgatc gatcccggca tcggccgttt 10020
tctctaccgc ctggcacgcc gcgccgcagg caaggcagaa gccagatggt tgttcaagac 10080
gatctacgaa cgcagtggca gcgccggaga gttcaagaag ttctgtttca ccgtgcgcaa 10140
gctgatcggg tcaaatgacc tgccggagta cgatttgaag gaggaggcgg ggcaggctgg 10200
cccgatccta gtcatgcgct accgcaacct gatcgagggc gaagcatccg ccggttccta 10260
atgtacggag cagatgctag ggcaaattgc cctagcaggg gaaaaaggtc gaaaaggtct 10320
ctttcctgtg gatagcacgt acattgggaa cccaaagccg tacattggga accggaaccc 10380
gtacattggg aacccaaagc cgtacattgg gaaccggtca cacatgtaag tgactgatat 10440
aaaagagaaa aaaggcgatt tttccgccta aaactcttta aaacttatta aaactcttaa 10500
aacccgcctg gcctgtgcat aactgtctgg ccagcgcaca gccgaagagc tgcaaaaagc 10560

gcctaccctt cggtcgctgc gctccctacg ccccgccgct tcgcgtcggc ctatcgcggc 10620
cgctggccgc tcaaaaatgg ctggcctacg gccaggcaat ctaccagggc gcggacaagc 10680
cgcgccgtcg ccactcgacc gccggcgccc acatcaaggc accggtgggt atgcctgacg 10740
atgcgtggag accgaaacct tgcgctcgtt cgccagccag gacagaaatg cctcgacttc 10800
gctgctgccc aaggttgccg ggtgacgcac accgtggaaa cggatgaagg cacgaaccca 10860
gtggacataa gcctgttcgg ttcgtaagct gtaatgcaag tagcgtatgc gctcacgcaa 10920
ctggtccaga accttgaccg aacgcagcgg tggtaacggc gcagtggcgg ttttcatggc 10980
ttgttatgac tgtttttttg gggtacagtc tatgcctcgg gcatccaagc agcaagcgcg 11040
ttacgccgtg ggtcgatgtt tgatgttatg gagcagcaac gatgttacgc agcagggcag 11100
tcgccctaaa acaaagttaa acatcatgag ggaagcggtg atcgccgaag tatcgactca 11160
actatcagag gtagttggcg tcatcgagcg ccatctcgaa ccgacgttgc tggccgtaca 11220
tttgtacggc tccgcagtgg atggcggcct gaagccacac agtgatattg atttgctggt 11280
tacggtgacc gtaaggcttg atgaaacaac gcggcgagct ttgatcaacg accttttgga 11340
aacttcggct tcccctggag agagcgagat tctccgcgct gtagaagtca ccattgttgt 11400
gcacgacgac atcattccgt ggcgttatcc agctaagcgc gaactgcaat ttggagaatg 11460
gcagcgcaat gacattcttg caggtatctt cgagccagcc acgatcgaca ttgatctggc 11520
tatcttgctg acaaaagcaa gagaacatag cgttgccttg gtaggtccag cggcggagga 11580
actctttgat ccggttcctg aacaggatct atttgaggcg ctaaatgaaa ccttaacgct 11640
atggaactcg ccgcccgact gggctggcga tgagcgaaat gtagtgctta cgttgtcccg 11700
catttggtac agcgcagtaa ccggcaaaat cgcgccgaag gatgtcgctg ccgactgggc 11760
aatggagcgc ctgccggccc agtatcagcc cgtcatactt gaagctagac aggcttatct 11820
tggacaagaa gaagatcgct tggcctcgcg cgcagatcag ttggaagaat ttgtccacta 11880
cgtgaaaggc gagatcacca aggtagtcgg caaataatgt ctaacaattc gttcaagccg 11940
acgccgcttc gcggcgcggc ttaactcaag cgttagatgc actaagcaca taattgctca 12000
cagccaaact atcaggtcaa gtctgctttt attattttta agcgtgcata ataagcccta 12060
cacaaattgg gagatatatc atgaaaggct ggctttttct tgttatcgca atagttggcg 12120
aagtaatcgc aacatagctt gcttggtcgt tccgcgtgaa cgtcggctcg attgtacctg 12180
cgttcaaata ctttgcgatc gtgttgcgcg cctgcccggt gcgtcggctg atctcacgga 12240
tcgactgctt ctctcgcaac gccatccgac ggatgatgtt taaaagtccc atgtggatca 12300
ctccgttgcc ccgtcgctca ccgtgttggg gggaaggtgc acatggctca gttctcaatg 12360
gaaattatct gcctaaccgg ctcagttctg cgtagaaacc aacatgcaag ctccaccggg 12420
tgcaaagcgg cagcggcggc aggatatatt caattgtaaa tggcttcatg tccgggaaat 12480
ctacatggat cagcaatgag tatgatggtc aatatggaga aaaagaaaga gtaattacca 12540
atttttttc aattcaaaaa tgtagatgtc cgcagcgtta ttataaaatg aaagtacatt 12600
ttgataaaac gacaaattac gatccgtcgt atttataggc gaaagcaata aacaaattat 12660
tctaattcgg aaatctttat ttcgacgtgt ctacattcac gtccaaatgg gggcttagat 12720
gagaaacttc acgatcggc 12739

<210> 2

<211> 11195
<212> DNA
<213> synthetic: expression vector - binary vector pSUNUbi-GFP

<400> 2
aatattcaaa caaacacata cagcgcgact tatcatggac atacaaatgg acgaacggat 60
aaaccttttc acgccctttt aaatatccga ttattctaat aaacgctctt ttctcttagg 120
tttacccgcc aatatatcct gtcaaacact gatagtttaa actgaaggcg ggaaacgaca 180
atcagatcta gtaggaaaca gctatgacca tgattacgcc aagctggccg cgtcgagcga 240

tctagtaaca tagatgacac cgcgcgcgat aatttatcct agtttgcgcg ctatattttg 300
ttttctatcg cgtattaaat gtataattgc gggactctaa tcataaaaac ccatctcata 360
aataacgtca tgcattacat gttaattatt acatgcttaa cgtaattcaa cagaaattat 420
atgataatca tcgcaagacc ggcaacagga ttcaatctta agaaacttta ttgccaaatg 480
tttgaacgat cggggaaatt cgagtcgact ctagaggatc cccggccgcc ccgactctag 540
tcgagttact tgtacagctc gtccatgccg agagtgatcc cggcggcggt cacgaactcc 600
agcaggacca tgtgatcgcg cttctcgttg gggtctttgc tcagggcgga ctgggtgctc 660
aggtagtggt tgtcgggcag cagcacgggg ccgtcgccga tgggggtgtt ctgctggtag 720
tggtcggcga gctgcacgct gccgtcctcg atgttgtggc gggtcttgaa gttcaccttg 780
atgccgttct tctgcttgtc ggcggtgata tagaccttgt ggctgttgta gttgtactcc 840
agcttgtgcc ccaggatgtt gccgtcctcc ttgaagtcga tgcccttcag ctcgatgcgg 900
ttcaccaggg tgtcgccctc gaacttcacc tcggcgcggg tcttgtagtt gccgtcgtcc 960
ttgaagaaga tggtgcgctc ctggacgtag ccttcgggca tggcggactt gaagaagtcg 1020
tgctgcttca tgtggtcggg gtagcgggcg aagcactgca cgccgtaggt caaggtggtc 1080
acgagggtgg gccagggcac gggcagcttg ccggtggtgc agatgaactt cagggtcagc 1140
ttgccgtagg tggcatcgcc ctcgccctcg ccggacacgc tgaacttgtg gccgtttacg 1200
tcgccgtcca gctcgaccag gatgggcacc accccggtga acagctcctc gcccttgctc 1260
accatggtgg ctttaccaac agtaccggaa tgccaagctt acttagatcg cagatctact 1320
agtatgatgg gtacccgggg gatccgatat cgcccgggct gcagaagtaa caccaaacaa 1380
cagggtgagc atcgacaaaa gaaacagtac caagcaaata aatagcgtat gaaggcaggg 1440
ctaaaaaaat ccacatatag ctgctgcata tgccatcatc caagtatatc aagatcaaaa 1500
taattataaa acatacttgt ttattataat agataggtac tcaaggttag agcatatgaa 1560
tagatgctgc atatgccatc atgtatatgc atcagtaaaa cccacatcaa catgtatacc 1620
tatcctagat cgatatttcc atccatctta aactcgtaac tatgaagatg tatgacacac 1680
acatacagtt ccaaaattaa taaatacacc aggtagtttg aaacagtatt ctactccgat 1740
ctagaacgaa tgaacgaccg cccaaccaca ccacatcatc acaaccaagc gaacaaaaag 1800
catctctgta tatgcatcag taaaacccgc atcaacatgt atacctatcc tagatcgata 1860
tttccatcca tcatcttcaa ttcgtaacta tgaatatgta tggcacacac atacagatcc 1920
aaaattaata aatccaccag gtagtttgaa acagaattct actccgatct agaacgaccg 1980
cccaaccaga ccacatcatc acaaccaaga caaaaaaaag catgaaaaga tgacccgaca 2040
aacaagtgca cggcatatat tgaaataaag gaaaagggca aaccaaaccc tatgcaacga 2100
aacaaaaaaa atcatgaaat cgatcccgtc tgcggaacgg ctagagccat cccaggattc 2160
cccaaagaga aacactggca agttagcaat cagaacgtgt ctgacgtaca ggtcgcatcc 2220
gtgtacgaac gctagcagca cggatctaac acaaacacgg atctaacaca aacatgaaca 2280
gaagtagaac taccgggccc taaccatgga ccggaacgcc gatctagaga aggtagagag 2340
gggggggggg ggaggacgag cggcgtacct tgaagcggag gtgccgacgg gtggatttgg 2400
gggagatctg gttgtgtgtg tgtgcgctcc gaacaacacg aggttgggga aagagggtgt 2460
ggagggggtg tctatttatt acggcgggcg aggaagggaa agcgaaggag cggtgggaaa 2520
ggaatccccc gtagctgccg tgccgtgaga ggaggaggag gccgcctgcc gtgccggctc 2580
acgtctgccg ctccgccacg caatttctgg atgccgacag cggagcaagt ccaacggtgg 2640
agcggaactc tcgagagggg tccagaggca gcgacagaga tgccgtgccg tctgcttcgc 2700
ttggcccgac gcgacgctgc tggttcgctg gttggtgtcc gttagactcg tcgacggcgt 2760
ttaacaggct ggcattatct actcgaaaca agaaaaatgt ttccttagtt tttttaattt 2820
cttaaagggt atttgtttaa tttttagtca ctttatttta ttctatttta tatctaaatt 2880
attaaataaa aaaactaaaa tagagtttta gttttcttaa tttagaggct aaaatagaat 2940
aaaatagatg tactaaaaaa attagtctat aaaaaccatt aaccctaaac cctaaatgga 3000
tgtactaata aaatggatga agtattatat aggtgaagct atttgcaaaa aaaaaggaga 3060
acacatgcac actaaaaaga taaaactgta gagtcctgtt gtcaaaatac tcaattgtcc 3120
tttagaccat gtctaactgt tcatttatat gattctctaa aacactgata ttattgtagt 3180

actatagatt atattattcg tagagtaaag tttaaatata tgtataaaga tagataaact 3240
gcacttcaaa caagtgtgac aaaaaaaata tgtggtaatt ttttataact tagacatgca 3300
atgctcatta tctctagaga ggggcacgac cgggtcacgc tgcactgcag gaattcgatg 3360
ggctcgaggt accgagctcg gtacccactg gattttggtt ttaggaatta gaaattttat 3420
tgatagaagt attttacaaa tacaaataca tactaagggt ttcttatatg ctcaacacat 3480
gagcgaaacc ctataagaac cctaattccc ttatctggga actactcaca cattattata 3540
gagagagata gatttgtaga gagagactgg tgatttcagc ggcatgcctg caggtcgact 3600
cagatctggg taactggcct aactggcctt ggaggagctg gcaactcaaa atccctttgc 3660
caaaaaccaa catcatgcca tccaccatgc ttgtatccag ctgcgcgcaa tgtaccccgg 3720
gctgtgtatc ccaaagcctc atgcaaccta acagatggat cgtttggaag gcctataaca 3780
gcaaccacag acttaaaacc ttgcgcctcc atagacttaa gcaaatgtgt gtacaatgtg 3840
gatcctaggc ccaacctttg atgcctatgt gacacgtaaa cagtactctc aactgtccaa 3900
tcgtaagcgt tcctagcctt ccagggccca gcgtaagcaa taccagccac aacaccctca 3960
acctcagcaa ccaaccaagg gtatctatct tgcaacctct ctagatcatc aatccactct 4020
tgtggtgttt gtggctctgt cctaaagttc actgtagacg tctcaatgta atggttaacg 4080
atatcacaaa ccgcggccat atcagctgct gtagctggcc taatctcaac tggtctcctc 4140
tccggagaca tgtcgactct agaggatccc ccgggctgca gaagtaacac caaacaacag 4200
ggtgagcatc gacaaaagaa acagtaccaa gcaaataaat agcgtatgaa ggcagggcta 4260
aaaaaatcca catatagctg ctgcatatgc catcatccaa gtatatcaag atcaaaataa 4320
ttataaaaca tacttgttta ttataataga taggtactca aggttagagc atatgaatag 4380
atgctgcata tgccatcatg tatatgcatc agtaaaaccc acatcaacat gtatacctat 4440
cctagatcga tatttccatc catcttaaac tcgtaactat gaagatgtat gacacacaca 4500
tacagttcca aaattaataa atacaccagg tagtttgaaa cagtattcta ctccgatcta 4560
gaacgaatga acgaccgccc aaccacacca catcatcaca accaagcgaa caaaaagcat 4620
ctctgtatat gcatcagtaa aacccgcatc aacatgtata cctatcctag atcgatattt 4680
ccatccatca tcttcaattc gtaactatga atatgtatgg cacacacata cagatccaaa 4740
attaataaat ccaccaggta gtttgaaaca gaattctact ccgatctaga acgaccgccc 4800
aaccagacca catcatcaca accaagacaa aaaaaagcat gaaaagatga cccgacaaac 4860
aagtgcacgg catatattga aataaaggaa aagggcaaac caaaccctat gcaacgaaac 4920
aaaaaaaatc atgaaatcga tcccgtctgc ggaacggcta gagccatccc aggattcccc 4980
aaagagaaac actggcaagt tagcaatcag aacgtgtctg acgtacaggt cgcatccgtg 5040
tacgaacgct agcagcacgg atctaacaca aacacggatc taacacaaac atgaacagaa 5100
gtagaactac cgggccctaa ccatggaccg gaacgccgat ctagagaagg tagagagggg 5160
ggggggggga ggacgagcgg cgtaccttga agcggaggtg ccgacgggtg gatttggggg 5220
agatctggtt gtgtgtgtgt gcgctccgaa caacacgagg ttggggaaag agggtgtgga 5280
gggggtgtct atttattacg gcgggcgagg aagggaaagc gaaggagcgg tgggaaagga 5340
atcccccgta gctgccgtgc cgtgagagga ggaggaggcc gcctgccgtg ccggctcacg 5400
tctgccgctc cgccacgcaa tttctggatg ccgacagcgg agcaagtcca acggtggagc 5460
ggaactctcg agaggggtcc agaggcagcg acagagatgc cgtgccgtct gcttcgcttg 5520
gcccgacgcg acgctgctgg ttcgctggtt ggtgtccgtt agactcgtcg acggcgttta 5580
acaggctggc attatctact cgaaacaaga aaaatgtttc cttagttttt ttaatttctt 5640
aaagggtatt tgtttaattt ttagtcactt tattttattc tattttatat ctaaattatt 5700
aaataaaaaa actaaaatag agttttagtt ttcttaattt agaggctaaa atagaataaa 5760
atagatgtac taaaaaaatt agtctataaa aaccattaac cctaaaccct aaatggatgt 5820
actaataaaa tggatgaagt attatatagg tgaagctatt tgcaaaaaaa aaggagaaca 5880
catgcacact aaaaagataa aactgtagag tcctgttgtc aaaatactca attgtccttt 5940
agaccatgtc taactgttca tttatatgat tctctaaaac actgatatta ttgtagtact 6000
atagattata ttattcgtag agtaaagttt aaatatatgt ataaagatag ataaactgca 6060
cttcaaacaa gtgtgacaaa aaaaatatgt ggtaattttt tataacttag acatgcaatg 6120

ctcattatct ctagagaggg gcacgaccgg gtcacgctgc actgcaggaa ttcgatatca 6180
agctggctga gtggctcctt caacgttgcg gttctgtcag ttccaaacgt aaaacggctt 6240
gtcccgcgtc atcggcgggg gtcataacgt gactccctta attctccgct catgatcaga 6300
ttgtcgtttc ccgccttcag tttaaactat cagtgtttga caggatcact gcttggtaat 6360
aattgtcatt agattgtttt tatgcataga tgcactcgaa atcagccaat tttagacaag 6420
tatcaaacgg atgttaattc agtacattaa agacgtccgc aatgtgttat taagttgtct 6480
aagcgtcaat ttgtttacac cacaatatat cctgccacca gccagccaac agctccccga 6540
ccggcagctc ggcacaaaat caccacgcgt ctaaaaaggt gatgtgtatt tgagtaaaac 6600
agcttgcgtc atgcggtcgc tgcgtatatg atgcgatgag taaataaaca aatacgcaag 6660
gggaacgcat gaaggttatc gctgtactta accagaaagg cgggtcaggc aagacgacca 6720
tcgcaaccca tctagcccgc gccctgcaac tcgccggggc cgatgttctg ttagtcgatt 6780
ccgatcccca gggcagtgcc cgcgattggg cggccgtgcg ggaagatcaa ccgctaaccg 6840
ttgtcggcat cgaccgcccg acgattgacc gcgacgtgaa ggccatcggc cggcgcgact 6900
tcgtagtgat cgacggagcg ccccaggcgg cggacttggc tgtgtccgcg atcaaggcag 6960
ccgacttcgt gctgattccg gtgcagccaa gcccttacga catatgggcc accgccgacc 7020
tggtggagct ggttaagcag cgcattgagg tcacggatgg aaggctacaa gcggcctttg 7080
tcgtgtcgcg ggcgatcaaa ggcacgcgca tcggcggtga ggttgccgag gcgctggccg 7140
ggtacgagct gcccattctt gagtcccgta tcacgcagcg cgtgagctac ccaggcactg 7200
ccgccgccgg cacaaccgtt cttgaatcag aacccgaggg cgacgctgcc cgcgaggtcc 7260
aggcgctggc cgctgaaatt aaatcaaaac tcatttgagt taatgaggta aagagaaaat 7320
gagcaaaagc acaaacacgc taagtgccgg ccgtccgagc gcacgcagca gcaaggctgc 7380
aacgttggcc agcctggcag acacgccagc catgaagcgg gtcaactttc agttgccggc 7440
ggaggatcac accaagctga agatgtacgc ggtacgccaa ggcaagacca ttaccgagct 7500
gctatctgaa tacatcgcgc agctaccaga gtaaatgagc aaatgaataa atgagtagat 7560
gaattttagc ggctaaagga ggcggcatgg aaaatcaaga acaaccaggc accgacgccg 7620
tggaatgccc catgtgtgga ggaacgggcg gttggccagg cgtaagcggc tgggttgtct 7680
gccggccctg caatggcact ggaaccccca agcccgagga atcggcgtga gcggtcgcaa 7740
accatccggc ccggtacaaa tcggcgcggc gctgggtgat gacctggtgg agaagttgaa 7800
ggccgcgcag gccgcccagc ggcaacgcat cgaggcagaa gacgccccgg tgaatcgtgg 7860
caaggggccg ctgatcgaat ccgcaaagaa tcccggcaac cgccggcagc cggtgcgccg 7920
tcgattagga agccgcccaa gggcgacgag caaccagatt ttttcgttcc gatgctctat 7980
gacgtgggca cccgcgatag tcgcagcatc atggacgtgg ccgttttccg tctgtcgaag 8040
cgtgaccgac gagctggcga ggtgatccgc tacgagcttc cagacgggca cgtagaggtt 8100
tccgcagggc cggccggcat ggccagtgtg tgggattacg acctggtact gatggcggtt 8160
tcccatctaa ccgaatccat gaaccgatac cgggaaggga agggagacaa gcccggccgc 8220
gtgttccgtc cacacgttgc ggacgtactc aagttctgcc ggcgagccga tggcggaaag 8280
cagaaagacg acctggtaga aacctgcatt cggttaaaca ccacgcacgt tgccatgcag 8340
cgtacgaaga aggccaagaa cggccgcctg gtgacggtat ccgagggtga agccttgatt 8400
agccgctaca agatcgtaaa gagcgaaacc gggcggccgg agtacatcga gatcgagcta 8460
gctgattgga tgtaccgcga gatcacagaa ggcaagaacc cggacgtgct gacggttcac 8520
cccgattact ttttgatcga tcccggcatc ggccgttttc tctaccgcct ggcacgccgc 8580
gccgcaggca aggcagaagc cagatggttg ttcaagacga tctacgaacg cagtggcagc 8640
gccggagagt tcaagaagtt ctgtttcacc gtgcgcaagc tgatcgggtc aaatgacctg 8700
ccggagtacg atttgaagga ggaggcgggg caggctggcc cgatcctagt catgcgctac 8760
cgcaacctga tcgagggcga agcatccgcc ggttcctaat gtacggagca gatgctaggg 8820
caaattgccc tagcagggga aaaaggtcga aaaggtctct ttcctgtgga tagcacgtac 8880
attgggaacc caaagccgta cattgggaac cggaacccgt acattgggaa cccaaagccg 8940
tacattggga accggtcaca catgtaagtg actgatataa aagagaaaaa aggcgatttt 9000
tccgcctaaa actctttaaa acttattaaa actcttaaaa cccgcctggc ctgtgcataa 9060

ctgtctggcc agcgcacagc cgaagagctg caaaaagcgc ctacccttcg gtcgctgcgc 9120
tccctacgcc ccgccgcttc gcgtcggcct atcgcggcct atgcggtgtg aaataccgca 9180
cagatgcgta aggagaaaat accgcatcag gcgctcttcc gcttcctcgc tcactgactc 9240
gctgcgctcg gtcgttcggc tgcggcgagc ggtatcagct cactcaaagg cggtaatacg 9300
gttatccaca gaatcagggg ataacgcagg aaagaacatg tgagcaaaag gccagcaaaa 9360
ggccaggaac cgtaaaaagg ccgcgttgct ggcgtttttc cataggctcc gcccccctga 9420
cgagcatcac aaaaatcgac gctcaagtca gaggtggcga aacccgacag gactataaag 9480
ataccaggcg tttccccctg gaagctccct cgtgcgctct cctgttccga ccctgccgct 9540
taccggatac ctgtccgcct ttctcccttc gggaagcgtg gcgctttctc atagctcacg 9600
ctgtaggtat ctcagttcgg tgtaggtcgt tcgctccaag ctgggctgtg tgcacgaacc 9660
ccccgttcag cccgaccgct gcgccttatc cggtaactat cgtcttgagt ccaacccggt 9720
aagacacgac ttatcgccac tggcagcagc cactggtaac aggattagca gagcgaggta 9780
tgtaggcggt gctacagagt tcttgaagtg gtggcctaac tacggctaca ctagaaggac 9840
agtatttggt atctgcgctc tgctgaagcc agttaccttc ggaaaaagag ttggtagctc 9900
ttgatccggc aaacaaacca ccgctggtag cggtggtttt tttgtttgca agcagcagat 9960
tacgcgcaga aaaaaaggat ctcaagaaga tcctttgatc ttttctacgg ggtctgacgc 10020
tcagtggaac gaaaactcac gttaagggat tttggtcatg catgatatat ctcccaattt 10080
gtgtagggct tattatgcac gcttaaaaat aataaaagca gacttgacct gatagtttgg 10140
ctgtgagcaa ttatgtgctt agtgcatcta acgcttgagt taagccgcgc cgcgaagcgg 10200
cgtcggcttg aacgaatttc tagctagaca ttatttgccg actaccttgg tgatctcgcc 10260
tttcacgtag tggacaaatt cttccaactg atctgcgcgc gaggccaagc gatcttcttc 10320
ttgtccaaga taagcctgtc tagcttcaag tatgacgggc tgatactggg ccggcaggcg 10380
ctccattgcc cagtcggcag cgacatcctt cggcgcgatt ttgccggtta ctgcgctgta 10440
ccaaatgcgg gacaacgtaa gcactacatt tcgctcatcg ccagcccagt cgggcggcga 10500
gttccatagc gttaaggttt catttagcgc ctcaaataga tcctgttcag gaaccggatc 10560
aaagagttcc tccgccgctg gacctaccaa ggcaacgcta tgttctcttg cttttgtcag 10620
caagatagcc agatcaatgt cgatcgtggc tggctcgaag atacctgcaa gaatgtcatt 10680
gcgctgccat tctccaaatt gcagttcgcg cttagctgga taacgccacg gaatgatgtc 10740
gtcgtgcaca acaatggtga cttctacagc gcggagaatc tcgctctctc caggggaagc 10800
cgaagtttcc aaaaggtcgt tgatcaaagc tcgccgcgtt gtttcatcaa gccttacggt 10860
caccgtaacc agcaaatcaa tatcactgtg tggcttcagg ccgccatcca ctgcggagcc 10920
gtacaaatgt acggccagca acgtcggttc gagatggcgc tcgatgacgc caactacctc 10980
tgatagttga gtcgatactt cggcgatcac cgcttccccc atgatgttta actttgtttt 11040
agggcgactg ccctgctgcg taacatcgtt gctgctccat aacatcaaac atcgacccac 11100
ggcgtaacgc gcttgctgct tggatgcccg aggcatagac tgtaccccaa aaaaacagtc 11160
ataacaagcc atgaaaaccg ccactgcgtt ccatg 11195

<210> 3

<211> 12415
<212> DNA
<213> synthetic: expression vector - binary vector pSUNUbiGUS

<400> 3
aatattcaaa caaacacata cagcgcgact tatcatggac atacaaatgg acgaacggat 60
aaaccttttc acgccctttt aaatatccga ttattctaat aaacgctctt ttctcttagg 120
tttacccgcc aatatatcct gtcaaacact gatagtttaa actgaaggcg ggaaacgaca 180
atcagatcta gtaggaaaca gctatgacca tgattacgcc aagcttgcat gcctgcaggt 240
cgaaattcga gctcggtacc cggtcactgg attttggttt taggaattag aaattttatt 300

gatagaagta ttttacaaat acaaatacat actaagggtt tcttatatgc tcaacacatg 360
agcgaaaccc tataagaacc ctaattccct tatctgggaa ctactcacac attattctgg 420
agaaaaatag agagagatag atttgtagag agagactggt gatttttgcg ccgggtaccg 480
agctcggtag caattcccga ggctgtagcc gacgatggtg cgccaggaga gttgttgatt 540
cattgtttgc ctccctgctg cggtttttca ccgaagttca tgccagtcca gcgtttttgc 600
agcagaaaag ccgccgactt cggtttgcgg tcgcgagtga agatcccttt cttgttaccg 660
ccaacgcgca atatgccttg cgaggtcgca aaatcggcga aattccatac ctgttcaccg 720
acgacggcgc tgacgcgatc aaagacgcgg tgatacatat ccagccatgc acactgatac 780
tcttcactcc acatgtcggt gtacattgag tgcagcccgg ctaacgtatc cacgccgtat 840
tcggtgatga taatcggctg atgcagtttc tcctgccagg ccagaagttc tttttccagt 900
accttctctg ccgtttccaa atcgccgctt tggacatacc atccgtaata acggttcagg 960
cacagcacat caaagagatc gctgatggta tcggtgtgag cgtcgcagaa cattacattg 1020
acgcaggtga tcggacgcgt cgggtcgagt ttacgcgttg cttccgccag tggcgaaata 1080
ttcccgtgca cttgcggacg ggtatccggt tcgttggcaa tactccacat caccacgctt 1140
gggtggtttt tgtcacgcgc tatcagctct ttaatcgcct gtaagtgcgc ttgctgagtt 1200
tccccgttga ctgcctcttc gctgtacagt tctttcggct tgttgcccgc ttcgaaacca 1260
atgcctaaag agaggttaaa gccgacagca gcagtttcat caatcaccac gatgccatgt 1320
tcatctgccc agtcgagcat ctcttcagcg taagggtaat gcgaggtacg gtaggagttg 1380
gccccaatcc agtccattaa tgcgtggtcg tgcaccatca gcacgttatc gaatcctttg 1440
ccacgtaagt ccgcatcttc atgacgacca aagccagtaa agtagaacgg tttgtggtta 1500
atcaggaact gttcgccctt cactgccact gaccggatgc cgacgcgaag cgggtagata 1560
tcacactctg tctggctttt ggctgtgacg cacagttcat agagataacc ttcacccggt 1620
tgccagaggt gcggattcac cacttgcaaa gtcccgctag tgccttgtcc agttgcaacc 1680
acctgttgat ccgcatcacg cagttcaacg ctgacatcac cattggccac cacctgccag 1740
tcaacagacg cgtggttaca gtcttgcgcg acatgcgtca ccacggtgat atcgtccacc 1800
caggtgttcg gcgtggtgta gagcattacg ctgcgatgga ttccggcata gttaaagaaa 1860
tcatggaagt aagactgctt tttcttgccg ttttcgtcgg taatcaccat tcccggcggg 1920
atagtctgcc agttcagttc gttgttcaca caaacggtga tacctgcaca tcaacaaatt 1980
ttggtcatat attagaaaag ttataaatta aaatatacac acttataaac tacagaaaag 2040
caattgctat atactacatt cttttatttt gaaaaaaata tttgaaatat tatattacta 2100
ctaattaatg ataattatta tatatatatc aaaggtagaa gcagaaactt acgtacactt 2160
ttcccggcaa taacatacgg cgtgacatcg gcttcaaatg gcgtatagcc gccctgatgc 2220
tccatcactt cctgattatt gacccacact ttgccgtaat gagtgaccgc atcgaaacgc 2280
agcacgatac gctggcctgc ccaacctttc ggtataaaga cttcgcgctg ataccagacg 2340
ttgcccgcat aattacgaat atctgcatcg gcgaactgat cgttaaaact gcctggcaca 2400
gcaattgccc ggctttcttg taacgcgctt tcccaccaac gctgaccaat tccacagttt 2460
tcgcgatcca gactgaatgc ccacaggccg tcgagttttt tgatttcacg ggttggggtt 2520
tctacaggac gtaccatggt cgatcgactc tagactagtg gatccgatat cgcccgggct 2580
gcagaagtaa caccaaacaa cagggtgagc atcgacaaaa gaaacagtac caagcaaata 2640
aatagcgtat gaaggcaggg ctaaaaaaat ccacatatag ctgctgcata tgccatcatc 2700
caagtatatc aagatcaaaa taattataaa acatacttgt ttattataat agataggtac 2760
tcaaggttag agcatatgaa tagatgctgc atatgccatc atgtatatgc atcagtaaaa 2820
cccacatcaa catgtatacc tatcctagat cgatatttcc atccatctta aactcgtaac 2880
tatgaagatg tatgacacac acatacagtt ccaaaattaa taaatacacc aggtagtttg 2940
aaacagtatt ctactccgat ctagaacgaa tgaacgaccg cccaaccaca ccacatcatc 3000
acaaccaagc gaacaaaaag catctctgta tatgcatcag taaaacccgc atcaacatgt 3060
atacctatcc tagatcgata tttccatcca tcatcttcaa ttcgtaacta tgaatatgta 3120
tggcacacac atacagatcc aaaattaata aatccaccag gtagtttgaa acagaattct 3180
actccgatct agaacgaccg cccaaccaga ccacatcatc acaaccaaga caaaaaaaag 3240

catgaaaaga tgacccgaca aacaagtgca cggcatatat tgaaataaag gaaaagggca 3300
aaccaaaccc tatgcaacga aacaaaaaaa atcatgaaat cgatcccgtc tgcggaacgg 3360
ctagagccat cccaggattc cccaaagaga aacactggca agttagcaat cagaacgtgt 3420
ctgacgtaca ggtcgcatcc gtgtacgaac gctagcagca cggatctaac acaaacacgg 3480
atctaacaca aacatgaaca gaagtagaac taccgggccc taaccatgga ccggaacgcc 3540
gatctagaga aggtagagag gggggggggg ggaggacgag cggcgtacct tgaagcggag 3600
gtgccgacgg gtggatttgg gggagatctg gttgtgtgtg tgtgcgctcc gaacaacacg 3660
aggttgggga aagagggtgt ggagggggtg tctatttatt acggcgggcg aggaagggaa 3720
agcgaaggag cggtgggaaa ggaatccccc gtagctgccg tgccgtgaga ggaggaggag 3780
gccgcctgcc gtgccggctc acgtctgccg ctccgccacg caatttctgg atgccgacag 3840
cggagcaagt ccaacggtgg agcggaactc tcgagagggg tccagaggca gcgacagaga 3900
tgccgtgccg tctgcttcgc ttggcccgac gcgacgctgc tggttcgctg gttggtgtcc 3960
gttagactcg tcgacggcgt ttaacaggct ggcattatct actcgaaaca agaaaaatgt 4020
ttccttagtt tttttaattt cttaaagggt atttgtttaa tttttagtca ctttatttta 4080
ttctatttta tatctaaatt attaaataaa aaaactaaaa tagagtttta gttttcttaa 4140
tttagaggct aaaatagaat aaaatagatg tactaaaaaa attagtctat aaaaaccatt 4200
aaccctaaac cctaaatgga tgtactaata aaatggatga agtattatat aggtgaagct 4260
atttgcaaaa aaaaaggaga acacatgcac actaaaaaga taaaactgta gagtcctgtt 4320
gtcaaaatac tcaattgtcc tttagaccat gtctaactgt tcatttatat gattctctaa 4380
aacactgata ttattgtagt actatagatt atattattcg tagagtaaag tttaaatata 4440
tgtataaaga tagataaact gcacttcaaa caagtgtgac aaaaaaaata tgtggtaatt 4500
ttttataact tagacatgca atgctcatta tctctagaga ggggcacgac cgggtcacgc 4560
tgcactgcag gaattcgatg ggctcgaggt accgagctcg gtacccactg gattttggtt 4620
ttaggaatta gaaattttat tgatagaagt attttacaaa tacaaataca tactaagggt 4680
ttcttatatg ctcaacacat gagcgaaacc ctataagaac cctaattccc ttatctggga 4740
actactcaca cattattata gagagagata gatttgtaga gagagactgg tgatttcagc 4800
ggcatgcctg caggtcgact cagatctggg taactggcct aactggcctt ggaggagctg 4860
gcaactcaaa atccctttgc caaaaaccaa catcatgcca tccaccatgc ttgtatccag 4920
ctgcgcgcaa tgtaccccgg gctgtgtatc ccaaagcctc atgcaaccta acagatggat 4980
cgtttggaag gcctataaca gcaaccacag acttaaaacc ttgcgcctcc atagacttaa 5040
gcaaatgtgt gtacaatgtg gatcctaggc ccaacctttg atgcctatgt gacacgtaaa 5100
cagtactctc aactgtccaa tcgtaagcgt tcctagcctt ccagggccca gcgtaagcaa 5160
taccagccac aacaccctca acctcagcaa ccaaccaagg gtatctatct tgcaacctct 5220
ctagatcatc aatccactct tgtggtgttt gtggctctgt cctaaagttc actgtagacg 5280
tctcaatgta atggttaacg atatcacaaa ccgcggccat atcagctgct gtagctggcc 5340
taatctcaac tggtctcctc tccggagaca tgtcgactct agaggatccc ccgggctgca 5400
gaagtaacac caaacaacag ggtgagcatc gacaaaagaa acagtaccaa gcaaataaat 5460
agcgtatgaa ggcagggcta aaaaaatcca catatagctg ctgcatatgc catcatccaa 5520
gtatatcaag atcaaaataa ttataaaaca tacttgttta ttataataga taggtactca 5580
aggttagagc atatgaatag atgctgcata tgccatcatg tatatgcatc agtaaaaccc 5640
acatcaacat gtatacctat cctagatcga tatttccatc catcttaaac tcgtaactat 5700
gaagatgtat gacacacaca tacagttcca aaattaataa atacaccagg tagtttgaaa 5760
cagtattcta ctccgatcta gaacgaatga acgaccgccc aaccacacca catcatcaca 5820
accaagcgaa caaaaagcat ctctgtatat gcatcagtaa aacccgcatc aacatgtata 5880
cctatcctag atcgatattt ccatccatca tcttcaattc gtaactatga atatgtatgg 5940
cacacacata cagatccaaa attaataaat ccaccaggta gtttgaaaca gaattctact 6000
ccgatctaga acgaccgccc aaccagacca catcatcaca accaagacaa aaaaaagcat 6060
gaaaagatga cccgacaaac aagtgcacgg catatattga aataaaggaa aagggcaaac 6120
caaaccctat gcaacgaaac aaaaaaaatc atgaaatcga tcccgtctgc ggaacggcta 6180

gagccatccc aggattcccc aaagagaaac actggcaagt tagcaatcag aacgtgtctg 6240
acgtacaggt cgcatccgtg tacgaacgct agcagcacgg atctaacaca aacacggatc 6300
taacacaaac atgaacagaa gtagaactac cgggccctaa ccatggaccg gaacgccgat 6360
ctagagaagg tagagagggg ggggggggga ggacgagcgg cgtaccttga agcggaggtg 6420
ccgacgggtg gatttggggg agatctggtt gtgtgtgtgt gcgctccgaa caacacgagg 6480
ttggggaaag agggtgtgga gggggtgtct atttattacg gcgggcgagg aagggaaagc 6540
gaaggagcgg tgggaaagga atcccccgta gctgccgtgc cgtgagagga ggaggaggcc 6600
gcctgccgtg ccggctcacg tctgccgctc cgccacgcaa tttctggatg ccgacagcgg 6660
agcaagtcca acggtggagc ggaactctcg agaggggtcc agaggcagcg acagagatgc 6720
cgtgccgtct gcttcgcttg gcccgacgcg acgctgctgg ttcgctggtt ggtgtccgtt 6780
agactcgtcg acggcgttta acaggctggc attatctact cgaaacaaga aaaatgtttc 6840
cttagttttt ttaatttctt aaagggtatt tgtttaattt ttagtcactt tattttattc 6900
tattttatat ctaaattatt aaataaaaaa actaaaatag agttttagtt ttcttaattt 6960
agaggctaaa atagaataaa atagatgtac taaaaaaatt agtctataaa aaccattaac 7020
cctaaaccct aaatggatgt actaataaaa tggatgaagt attatatagg tgaagctatt 7080
tgcaaaaaaa aaggagaaca catgcacact aaaaagataa aactgtagag tcctgttgtc 7140
aaaatactca attgtccttt agaccatgtc taactgttca tttatatgat tctctaaaac 7200
actgatatta ttgtagtact atagattata ttattcgtag agtaaagttt aaatatatgt 7260
ataaagatag ataaactgca cttcaaacaa gtgtgacaaa aaaaatatgt ggtaattttt 7320
tataacttag acatgcaatg ctcattatct ctagagaggg gcacgaccgg gtcacgctgc 7380
actgcaggaa ttcgatatca agctggctga gtggctcctt caacgttgcg gttctgtcag 7440
ttccaaacgt aaaacggctt gtcccgcgtc atcggcgggg gtcataacgt gactccctta 7500
attctccgct catgatcaga ttgtcgtttc ccgccttcag tttaaactat cagtgtttga 7560
caggatcact gcttggtaat aattgtcatt agattgtttt tatgcataga tgcactcgaa 7620
atcagccaat tttagacaag tatcaaacgg atgttaattc agtacattaa agacgtccgc 7680
aatgtgttat taagttgtct aagcgtcaat ttgtttacac cacaatatat cctgccacca 7740
gccagccaac agctccccga ccggcagctc ggcacaaaat caccacgcgt ctaaaaaggt 7800
gatgtgtatt tgagtaaaac agcttgcgtc atgcggtcgc tgcgtatatg atgcgatgag 7860
taaataaaca aatacgcaag gggaacgcat gaaggttatc gctgtactta accagaaagg 7920
cgggtcaggc aagacgacca tcgcaaccca tctagcccgc gccctgcaac tcgccggggc 7980
cgatgttctg ttagtcgatt ccgatcccca gggcagtgcc cgcgattggg cggccgtgcg 8040
ggaagatcaa ccgctaaccg ttgtcggcat cgaccgcccg acgattgacc gcgacgtgaa 8100
ggccatcggc cggcgcgact tcgtagtgat cgacggagcg ccccaggcgg cggacttggc 8160
tgtgtccgcg atcaaggcag ccgacttcgt gctgattccg gtgcagccaa gcccttacga 8220
catatgggcc accgccgacc tggtggagct ggttaagcag cgcattgagg tcacggatgg 8280
aaggctacaa gcggcctttg tcgtgtcgcg ggcgatcaaa ggcacgcgca tcggcggtga 8340
ggttgccgag gcgctggccg ggtacgagct gcccattctt gagtcccgta tcacgcagcg 8400
cgtgagctac ccaggcactg ccgccgccgg cacaaccgtt cttgaatcag aacccgaggg 8460
cgacgctgcc cgcgaggtcc aggcgctggc cgctgaaatt aaatcaaaac tcatttgagt 8520
taatgaggta aagagaaaat gagcaaaagc acaaacacgc taagtgccgg ccgtccgagc 8580
gcacgcagca gcaaggctgc aacgttggcc agcctggcag acacgccagc catgaagcgg 8640
gtcaactttc agttgccggc ggaggatcac accaagctga agatgtacgc ggtacgccaa 8700
ggcaagacca ttaccgagct gctatctgaa tacatcgcgc agctaccaga gtaaatgagc 8760
aaatgaataa atgagtagat gaattttagc ggctaaagga ggcggcatgg aaaatcaaga 8820
acaaccaggc accgacgccg tggaatgccc catgtgtgga ggaacgggcg gttggccagg 8880
cgtaagcggc tgggttgtct gccggccctg caatggcact ggaaccccca agcccgagga 8940
atcggcgtga gcggtcgcaa accatccggc ccggtacaaa tcggcgcggc gctgggtgat 9000
gacctggtgg agaagttgaa ggccgcgcag gccgcccagc ggcaacgcat cgaggcagaa 9060
gacgccccgg tgaatcgtgg caaggggccg ctgatcgaat ccgcaaagaa tcccggcaac 9120

cgccggcagc cggtgcgccg tcgattagga agccgcccaa gggcgacgag caaccagatt 9180
ttttcgttcc gatgctctat gacgtgggca cccgcgatag tcgcagcatc atggacgtgg 9240
ccgttttccg tctgtcgaag cgtgaccgac gagctggcga ggtgatccgc tacgagcttc 9300
cagacgggca cgtagaggtt tccgcagggc cggccggcat ggccagtgtg tgggattacg 9360
acctggtact gatggcggtt tcccatctaa ccgaatccat gaaccgatac cgggaaggga 9420
agggagacaa gcccggccgc gtgttccgtc cacacgttgc ggacgtactc aagttctgcc 9480
ggcgagccga tggcggaaag cagaaagacg acctggtaga aacctgcatt cggttaaaca 9540
ccacgcacgt tgccatgcag cgtacgaaga aggccaagaa cggccgcctg gtgacggtat 9600
ccgagggtga agccttgatt agccgctaca agatcgtaaa gagcgaaacc gggcggccgg 9660
agtacatcga gatcgagcta gctgattgga tgtaccgcga gatcacagaa ggcaagaacc 9720
cggacgtgct gacggttcac cccgattact ttttgatcga tcccggcatc ggccgttttc 9780
tctaccgcct ggcacgccgc gccgcaggca aggcagaagc cagatggttg ttcaagacga 9840
tctacgaacg cagtggcagc gccggagagt tcaagaagtt ctgtttcacc gtgcgcaagc 9900
tgatcgggtc aaatgacctg ccggagtacg atttgaagga ggaggcgggg caggctggcc 9960
cgatcctagt catgcgctac cgcaacctga tcgagggcga agcatccgcc ggttcctaat 10020
gtacggagca gatgctaggg caaattgccc tagcagggga aaaaggtcga aaaggtctct 10080
ttcctgtgga tagcacgtac attgggaacc caaagccgta cattgggaac cggaacccgt 10140
acattgggaa cccaaagccg tacattggga accggtcaca catgtaagtg actgatataa 10200
aagagaaaaa aggcgatttt tccgcctaaa actctttaaa acttattaaa actcttaaaa 10260
cccgcctggc ctgtgcataa ctgtctggcc agcgcacagc cgaagagctg caaaaagcgc 10320
ctacccttcg gtcgctgcgc tccctacgcc ccgccgcttc gcgtcggcct atcgcggcct 10380
atgcggtgtg aaataccgca cagatgcgta aggagaaaat accgcatcag gcgctcttcc 10440
gcttcctcgc tcactgactc gctgcgctcg gtcgttcggc tgcggcgagc ggtatcagct 10500
cactcaaagg cggtaatacg gttatccaca gaatcagggg ataacgcagg aaagaacatg 10560
tgagcaaaag gccagcaaaa ggccaggaac cgtaaaaagg ccgcgttgct ggcgtttttc 10620
cataggctcc gcccccctga cgagcatcac aaaaatcgac gctcaagtca gaggtggcga 10680
aacccgacag gactataaag ataccaggcg tttccccctg gaagctccct cgtgcgctct 10740
cctgttccga ccctgccgct taccggatac ctgtccgcct ttctcccttc gggaagcgtg 10800
gcgctttctc atagctcacg ctgtaggtat ctcagttcgg tgtaggtcgt tcgctccaag 10860
ctgggctgtg tgcacgaacc ccccgttcag cccgaccgct gcgccttatc cggtaactat 10920
cgtcttgagt ccaacccggt aagacacgac ttatcgccac tggcagcagc cactggtaac 10980
aggattagca gagcgaggta tgtaggcggt gctacagagt tcttgaagtg gtggcctaac 11040
tacggctaca ctagaaggac agtatttggt atctgcgctc tgctgaagcc agttaccttc 11100
ggaaaaagag ttggtagctc ttgatccggc aaacaaacca ccgctggtag cggtggtttt 11160
tttgtttgca agcagcagat tacgcgcaga aaaaaaggat ctcaagaaga tcctttgatc 11220
ttttctacgg ggtctgacgc tcagtggaac gaaaactcac gttaagggat tttggtcatg 11280
catgatatat ctcccaattt gtgtagggct tattatgcac gcttaaaaat aataaaagca 11340
gacttgacct gatagtttgg ctgtgagcaa ttatgtgctt agtgcatcta acgcttgagt 11400
taagccgcgc cgcgaagcgg cgtcggcttg aacgaatttc tagctagaca ttatttgccg 11460
actaccttgg tgatctcgcc tttcacgtag tggacaaatt cttccaactg atctgcgcgc 11520
gaggccaagc gatcttcttc ttgtccaaga taagcctgtc tagcttcaag tatgacgggc 11580
tgatactggg ccggcaggcg ctccattgcc cagtcggcag cgacatcctt cggcgcgatt 11640
ttgccggtta ctgcgctgta ccaaatgcgg gacaacgtaa gcactacatt tcgctcatcg 11700
ccagcccagt cgggcggcga gttccatagc gttaaggttt catttagcgc ctcaaataga 11760
tcctgttcag gaaccggatc aaagagttcc tccgccgctg gacctaccaa ggcaacgcta 11820
tgttctcttg cttttgtcag caagatagcc agatcaatgt cgatcgtggc tggctcgaag 11880
atacctgcaa gaatgtcatt gcgctgccat tctccaaatt gcagttcgcg cttagctgga 11940
taacgccacg gaatgatgtc gtcgtgcaca acaatggtga cttctacagc gcggagaatc 12000
tcgctctctc caggggaagc cgaagtttcc aaaaggtcgt tgatcaaagc tcgccgcgtt 12060

gtttcatcaa gccttacggt caccgtaacc agcaaatcaa tatcactgtg tggcttcagg 12120
ccgccatcca ctgcggagcc gtacaaatgt acggccagca acgtcggttc gagatggcgc 12180
tcgatgacgc caactacctc tgatagttga gtcgatactt cggcgatcac cgcttccccc 12240
atgatgttta actttgtttt agggcgactg ccctgctgcg taacatcgtt gctgctccat 12300
aacatcaaac atcgacccac ggcgtaacgc gcttgctgct tggatgcccg aggcatagac 12360
tgtaccccaa aaaaacagtc ataacaagcc atgaaaaccg ccactgcgtt ccatg 12415

## Description

### BACKGROUND OF THE INVENTION

### Field of the Invention

The present invention relates to methods for the incorporation of DNA into the genome of a Gramineae plant, preferably a wheat plant, by means of Agrobacterium-mediated transformation. In other aspects, the invention relates to the production of stably transformed and fertile Gramineae plants, preferably wheat plants, gametes and offspring from these plants.

### Description of the Related Art

During the past decade, it has become possible to transfer genes from a wide range of organisms to crop plants by recombinant DNA technology. This advance has provided enormous opportunities to improve plant resistance to pests, diseases and herbicides, and to modify biosynthetic processes to change the quality of plant products. However, the availability of an efficient transformation method to introduce foreign DNA has been a substantial barrier for most monocot species, including maize, rice, oat, barley, and particularly wheat.

There have been many methods attempted for the transformation of monocotyledonous plants, wherein "biolistics" is the most widely used transformation method for monocotyledons. In the "biolistics" (microprojectile-mediated DNA delivery) method microprojectile particles are coated with DNA and accelerated by a mechanical device to a speed high enough to penetrate the plant cell wall and nucleus (WO 91/02071). The foreign DNA gets incorporated into the host DNA and results in a transformed cell. There are many variations on the "biolistics" method (Sanford, 1990; Fromm et al., 1990; Christou et al., 1988; Sautter et al., 1991). The method has been used to produce stably transformed monocotyledonous plants including rice, maize, wheat, barley, and oats (Christou et al., 1991; Gordon-Kamm et al., 1990; Vasil et at., 1992, 1993; Wan et al., 1994; Sommers et al., 1992). However, even with the more recent improvements in transformation methods using immature embryos as target tissues, it still requires 4 to 6 months to recover transgenic plants (Weeks et al., 1993; Vasil et al., 1992; 1993; Becker et al., 1994, Rasco-Gaunt et al. 2001). The frequency of transgenic plant formation by these methods is variable ranging from less than one to more than 17 events from 100 bombarded embryos (Rasco-Gaunt et al. 2001). Microprojectile-mediated DNA delivery brings about a number of problems such as frequent fragmentation of the DNA-sequence prior to its integration, random integration in transcribed as well as non-transcribed chromosomal regions, predominantly multiple insertion of the sequence to be transferred, complex integration patterns, integeration of backbone sequences including selectable marker genes at the same locus. Moreover, microprojectile-mediated plant transformation is generally based upon genotype-dependend cell culture methods which often require a secondary transfer of the transgene into the background of elite beeding material via long-lasting back-crossing.

Protoplast based methods have been used mostly in rice, where DNA is delivered to the protoplasts through liposomes, PEG, or electroporation (Shimamoto 1989; Datta 1990b). Protoplasts may be isolated from various tissues but require in general the use of cell wall-degrading enzymes. It is considered likely that the use of cell wall-degrading enzymes can inhibit the subsequent regeneration process. Furthermore, most protoplast based methods require the establishment of long-term embryogenic suspension cultures. Some regenerants from protoplasts are infertile and phenotypically abnormal due to somaclonal variation during the long-term suspension culture (Davey 1991; Rhodes 1988). Transformation by electroporation involves the application of short, high-voltage electric fields to create "pores" in the cell membrane through which DNA is taken-up. This method has been used to produce stably transformed monocotyledonous plants (Paszkowski 1984; Shillito 1985; Fromm 1986) especially from rice (Shimamoto 1989; Datta 1990b; Hayakawa 1992).

A number of other methods have been reported for the transformation of monocotyledonous plants including, for example, the "pollen tube method" (WO 93/18168; Luo 1988), macro-injection of DNA into floral tillers (Du 1989; De la Pena 1987), injection of Agrobacterium into developing caryopses (WO 00/63398), and tissue incubation of seeds in DNA solutions (Töpfer et al. 1989). Direct injection of exogenous DNA into the fertilized plant ovule at the onset of embryogenesis was disclosed in WO 94/00583.

While widely useful in dicotyledonous plants, Agrobacterium-mediated gene transfer has long been disappointing when adapted to use in monocots. There are several reports in the literature claiming Agrobacterium transformation of monocotyledons (e.g, discussed WO 94/00977). These are specifically the methods of Gould 1991; Mooney 1991; and Raineri 1990, which claim Agrobacterium transformation of maize, rice and wheat. There is some evidence of gene transfer in these methods but they lack convincing evidence for transfer efficiency, reproducibility, and confirmation of gene transfer (Potrykus, 1990), and lack of transfer to the progeny when plants are produced. In the work of Gould where evidence of transformed plants was presented there was no Mendelian inheritance of the genes. Attempts by Hiei et al. (1994) suggested that transgenic rice plants could be obtained following Agrobacterium-mediated transformation, but the particular bacterial strains used and the choice of bacterial vectors were critical for successfully obtaining transgenics. A paper by Ishida et al. (1996) indicated that high-effciency transformation of maize was possible by co-culture of immature embryos with A. tumefaciens. In both reports on rice and maize transformation, a super-binary vector pTOK233 containing additional copies of the virB, virC and virG genes was used to achieve high-efficiency transformation. A report by Saito et al. (WO 95/06722) disclosed a method of transforming monocotyledons using scutellum of immature embryos with A. tumefaciens.

WO 97/48814 disclosed a process for producing stably transformed fertile wheat and a system of transforming wheat via Agrobacterium based on freshly isolated or pre-cultured immature embryos, embryogenic callus and suspension cells. Also disclosed are methods for recovering transgenic plants after transformation within a short period of time, provided the explants are regenerable at the time of transformation. All methods disclosed therein use at some stage dedifferentiation of the target tissue. Selection is performed at callus stage to enable preferential development of transformed tissue eventually resulting in regeneration of homogenously transformed plants. The number of transgenic events obtained from the various starting materials is relatively low and generally below the 1% transformation efficacy range. This makes any effort to obtain transgenic wheat plants a laborious, cost-intensive approach.

The above mentioned plant transformation systems involve callus-based selection protocols and extensive in vitro culturing. These methodologies are time-consuming and increase the likelihood that somaclonal variants will arise that exhibit undesirable agronomic characteristics. Furthermore, these methods are extremely genotype-depending and need extensive adoption of the cell culture conditions for each plant species (and often even each line). Use of developmentally organized explants (such as immature embryos) as targets for transformation circumvent time-consuming tissue culture steps but increases the likelihood that chimeric plants are produced.

Targeting gametes, zygotes or early stage embryos in embryo sacs for transformation is a potential solution to these problems. WO 98/01576 discloses a method of plant (especially maize) transformation in which a foreign gene might be introduced into the zyogte situated in its natural cellular context (the embryo sac). The intact embryo sac is claimed to be of essential importance for the subsequent recovery of transgenic plants. While Agrobacterium-mediated transformation of those in situ maize zygotes is hypothetically described, it seems unlikely that this approach will result in stably transformed plants, since Agrobacteria have only severely restricted access to the zygote within the embryo sac.

The differentiation of the zygote into an embryo capable of germination is a key process in the life cycle of plants. Investigations on embryogenesis in higher plants have been performed by either descriptive examination of morphology (reviewed by Johri et al. 1992), genetic analysis of developmental mutants (reviewed by Meinke 1993 and by Jürgens et al. 1994), or molecular analysis of gene expression (Thomas 1993). Although the production of zygotes by plants is well-understood, reproducible methods for in vitro manipulation and transformation of these cells are needed. In most plant species zygotes are comprised in embryo sacs which are deeply enclosed in the sporophytic tissues and therefore the zygotes are difficult to manipulate. Pollination involves the transfer of pollen from anther to stigma. Pollen germinates on the stigma and a pollen tube grows through the style and enters the tip of the ovule through the micropyle. Two male gametes or sperm cells are formed by division of the generative cell of the pollen grain. The sperm cells move through the pollen tube and are emptied into the embryo sac. Within each ovule there is a megasporocyte that undergoes meiosis to generate four haploid megaspores. Three of the megaspores usually disintegrate while the fourth continues to undergo divisions and forms an embryo sac which originally contains six haploid cells as well as the diploid central cell. The egg and two synergids are found near the micropyle while three antipodals are found at the chalazal end of the embryo sac. One sperm cell fuses with the egg cell to form the zygote which eventually develops into the embryo and a new plant. The second sperm fuses with the central cell to form the triploid primary endosperm cell which constitutes the origine of the endosperm.

Methods for the isolation of living embryo sacs have been developed for plant species by using either micro-dissection or enzymatic digestion (Allington, 1985; Theunis 1991; Wu 1993). In vitro manipulation of fertilized embryo sacs frequently results in low viability or production of abnormal embryos (Leduc 1995). To date, only a few reports have been issued on embryonic development and regeneration from higher-plant zygotes free from maternal tissue. Kranz and coworkers reported microcallus development (Kranz et al. 1991; Kranz and Lörz 1994), embryogenesis and plant regeneration (Kranz and Lörz 1993) from cultivated maize zygotes which had been produced by in-vitro fertilization using isolated sperm and egg cells. Formation of embryo-like structures followed by plant regeneration was also reproducibly obtained by cultivating isolated barley (Holm et al. 1994) and maize zygotes (Leduc et al. 1996) after fertilization in planta. Furthermore, methods for isolation of wheat zygotes and their regeneration into wheat plants are described (Kumlehn et al. 1997; Kumlehn et al. 1998).

WO 94/01999 is describing a method of regeneration of plants from fertilized, isolated egg protoplasts. Furthermore, a method of transformation of fertilized egg cells and regeneration of transgenic plants therefrom is described. It is explicitly stated, that grasses (Gramineae like e.g., barley or wheat) are not susceptible to Agrobacterium-mediated transformation (with reference to Potrykus, 1991). The employed transformation method is therefore restricted to microinjection of DNA into the isolated egg cell.

Agrobacterium-mediated transformation of isolated zygotes may appear to be hardly to manage, since there have so far been some unresolved technical requirements: The active release of signal molecules from the feeder-system which is indispensible for zygote survival and development has to be ensured throughout co-culture of zygotes and Agrobacteria and thereafter. While the zygotes are infected with Agrobacteria, the Agrobacteria should be kept apart from the co-cultured feeder cells. On the other hand, signal molecules from the feeder tissue have to have access to the isolated zygotes. Moreover, washing off the Agrobacteria from the extremely sensitive zygotes or zygote-derived structures and the transfer of the target cells to fresh medium after co-culture would undoubtly cause severe cell damage or even collapse of the plasma membrane. After co-culture, these objects need to be transferred to an Agrobacterium-free medium which has exactly the same osmolality as the co-culture medium used before. Since the osmolality of the medium depends on the uptake of nutrients and the release of substances from the cells, after a few days practically every culture has its particular osmolality which is hardly reproducible in another medium containing actively developing feeder cells.

Despite the fact that wheat is the most widely-grown cereal crop in the world, unfortunately no efficient, cost-effective and genotype-independend method exists on the use of Agrobacterium transformation to generate stable, fertile transgenic wheat plants. A need therefore exists for an efficient method to genetically transform wheat genotypes of choice, preferentially elite breeding lines, the transgenic plants being uniformly transformed with a foreign gene. A need also exists for an efficient method for the production of transformed wheat plants capable of transmitting a foreign gene to progeny. In addition, a need exists for a method for Agrobacterium-mediated transformation of isolated wheat zygotes and subsequent generation of transgenic wheat plants. An additional need (especially in the light of regulatory requirements) exists for a transformation method which is independent of the use of a selectable marker gene (like e.g., antibiotic or herbicide resitance). Further, a technical solution is required to prevent the co-cultured feeder cells from being infected during co-culture of zygotes and Agrobacteria as well as to ensure transfer of the zygotes or zygote-derived structures to Agrobacterium-free, equiosmolar medium after co-culture.

Therefore, it is an objective of the current invention to provide a genotype-independend, efficient method for generating fertile, transgenic wheat plants.

### SUMMARY OF THE INVENTION

It is a particular object of the present invention to provide techniques that will allow to prepare transgenic, fertile Gramineae plants, which have been stably transformed through the introduction of one or more desired genes into the genome of these species. Accordingly a first embodiment of the invention is related to a method for producing a transgenic Gramineae plant comprising the steps of:
(a) isolating a zygote from a Gramineae plant to be transformed in a way that said isolated zygote becomes substantially free from its naturally surrounding tissue,
(b) introducing a DNA composition comprising a genetic component into the genome of said Gramineae plant, wherein said introduction is mediated by Agrobacterium transformation into said isolated zygote;
(c) regenerating Gramineae plants from said zygotes which have received said genetic component; and
(d) identifying a fertile, transgenic Gramineae plant whose genome has been altered through the stable introduction of said genetic component.

In a preferred embodiment, said Gramineae plant is selected from the group consisting of wheat, maize and barley. More preferably, the Gramineae plant is a Triticum species.

In another preferred embodiment, the regeneration step (c) of the method of the invention comprises a step, wherein the Gramineae plant is regenerated from said isolated zygote by a method comprising co-cultivation of said isolated zygote and/or zygotic embryo derived therefrom with immature pollen, a cell culture derived from immature pollen, or a culture comprising cells derived from immature pollen. Preferably, the co-cultivation is carried out using a culture of isolated, androgenetically developing immature barley pollen.

In another preferred embodiment of the invention, the genetic component is transmitted through a complete sexual cycle of said transgenic Gramineae plant to its progeny.

Because of the high efficiency of the method of the invention, no selection or screening step based on selectable or screenable markers is necessary. The identification of transgenic Gramineae plants obtained by the method of the invention can be carried out simply by screening for the genetic component introduced into the genome of said Gramineae plant (e.g., by means of PCR or Southern blotting). Therefore, in another preferred embodiment, the transgenic Gramineae plant obtained by the method of the invention and/or the progeny thereof does not comprise a selectable or screenable marker gene.

In another preferred embodiment of the invention, the method does not comprise any step which leads to dedifferentiation of the zygote or the zygote-derived embryo. More preferably, no auxine or cytokinin compounds are utilized in a concentration and/or combination which leads to dedifferentiation.

In another preferred embodiment the genetic component introduced into the genome of said Gramineae plant comprises an expression cassette comprising a nucleic acid sequence operably linked to a promoter active in said Gramineae plant, wherein expression of said nucleic acid sequence confers a phenotypically distinguishable trait to said Gramineae plant.

### BRIEF DESCRIPTION OF THE DRAWINGS

The drawings form part of the present specification and are included to further demonstrate certain aspects of the present invention. The invention may be better understood by reference to these drawings in combination with the detailed description of specific embodiments presented herein.

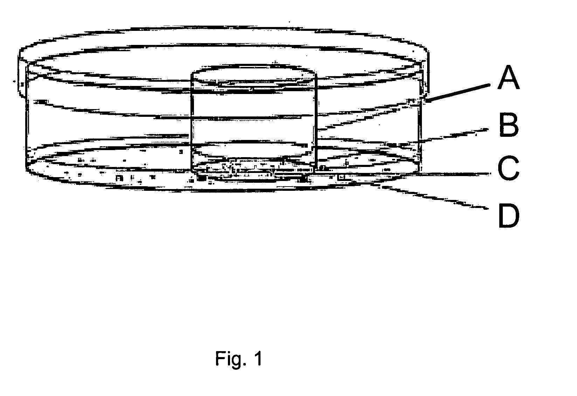

- Fig.1:: A: Millicell insert;
B: semi-permeable membrane;
C: isolated zygotes (or co-culture of zygotes and Agrobacteria)
D: feeder system (e.g., androgenically developing pollen culture of barley)

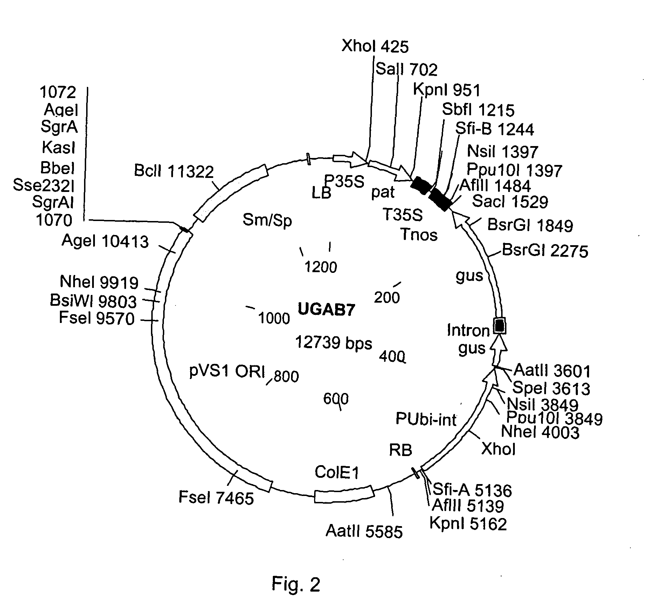

- Fig. 2:: Plasmid map of pUGAB7 (binary vector comprising β-Glucuronidase encoding sequence und control of the maize ubiquitin promoter). PUbi-int: Maize ubiquitin 1 promoter comprising intron 1; GUS: β-Glucuronidase encoding sequence; P35S: CaMV 35S promoter; pat: phosphinotricinacetyltransferase (BASTA™ resistance gene); T35S: terminator sequence of CaMV 35S gene; Tnos: NOS terminator; LB/RB: left/right border of Agrobacterium T-DNA; Sm/Sp: Spectinomycin resistance gene; colE1: origin of replication; pVS1 ORI: origin of replication. Important restriction enzyme sites are indicated by name of the corresponding enzyme and the restriction site by base pair number.

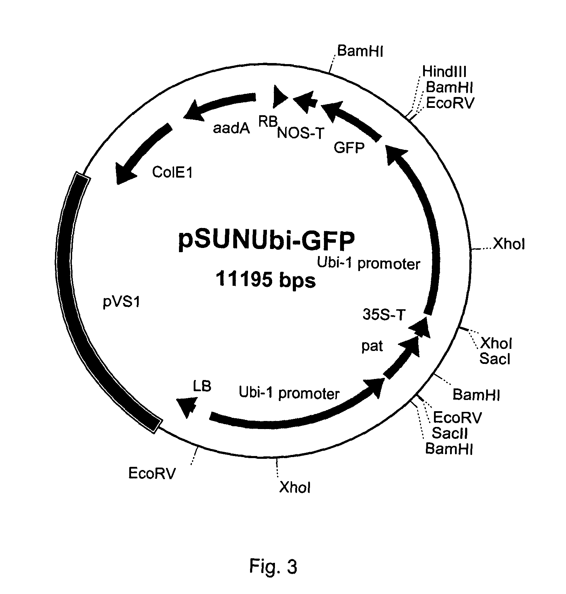

- Fig. 3:: Plasmid map of pSUN-Ubi-GFP (binary vector comprising green fluorescence protein encoding sequence und control of the maize ubiquitin promoter). Ubi-1: Maize ubiquitin 1 promoter; GFP: green fluorescence protein encoding sequence; pat: phosphinotricinacetyltransferase (BASTA™ resistance gene); 35S-T: terminator sequence of CaMV 35S gene; nos-T: NOS terminator; LB/RB: left/right border of Agrobacterium T-DNA; aadA: Spectinomycin resistance gene; colE1: origin of replication. Important restriction enzyme sites are indicated by name of the corresponding enzyme.

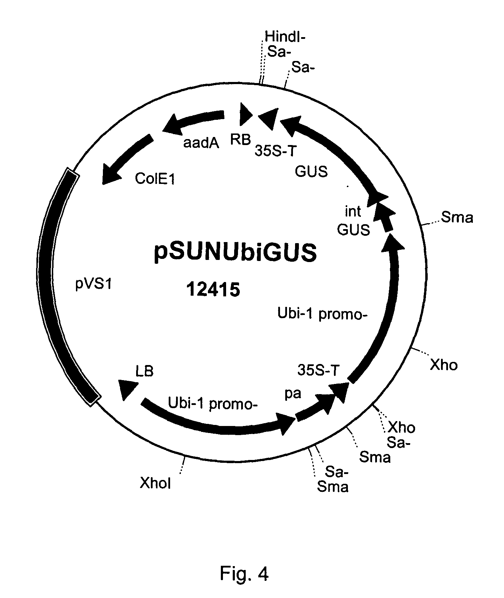

- Fig. 4:: Plasmid map of pSUN-Ubi-GUS (binary vector comprising β-Glucuronidase encoding sequence und control of the maize ubiquitin promoter). Ubi-1: Maize ubiquitin 1 promoter; GUS: β-Glucuronidase encoding sequence; pat: phosphinotricinacetyltransferase (BASTA™ resistance gene); 35S-T: terminator sequence of CaMV 35S gene; nos-T: NOS terminator; LB/RB: left/right border of Agrobacterium T-DNA; aadA: Spectinomycin resistance gene; colE1: origin of replication. Important restriction enzyme sites are indicated by name of the corresponding enzyme.

### GENERAL DEFINITIONS

It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent up or down (higher or lower).

As used herein, the word "or" means any one member of a particular list and also includes any combination of members of that list.

The term "nucleic acid" refers to deoxyribonucleotides or ribonucleotides and polymers or hybrids thereof in either single-or double-stranded, sense or antisense form.

Unless otherwise indicated, a particular nucleic acid sequence also implicitly encompasses conservatively modified variants thereof (e. g., degenerate codon substitutions) and complementary sequences, as well as the sequence explicitly indicated. The term "nucleic acid" is used interchangeably herein with "gene", "cDNA, "mRNA", "oligonucleotide," and "polynucleotide".

The phrase "nucleic acid sequence" as used herein refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest. A "coding region" of a nucleic acid is the portion of the nucleic acid which is transcribed and translated in a sequence-specific manner to produce into a particular polypeptide or protein when placed under the control of appropriate regulatory sequences. The coding region is said to encode such a polypeptide or protein.

The term "antisense" is understood to mean a nucleic acid having a sequence complementary to a target sequence, for example a messenger RNA (mRNA) sequence the blocking of whose expression is sought to be initiated by hybridization with the target sequence.

The term "sense" is understood to mean a nucleic acid having a sequence which is homologous or identical to a target sequence, for example a sequence which binds to a protein transcription factor and which is involved in the expression of a given gene. According to a preferred embodiment, the nucleic acid comprises a gene of interest and elements allowing the expression of the said gene of interest.

The term "gene" refers to a coding region operably joined to appropriate regulatory sequences capable of regulating the expression of the polypeptide in some manner. A gene includes untranslated regulatory regions of DNA (e. g., promoters, enhancers, repressors, etc.) preceding (upstream) and following (downstream) the coding region (open reading frame, ORF) as well as, where applicable, intervening sequences (i.e., introns) between individual coding regions (i.e., exons).

As used herein the term "coding region" when used in reference to a structural gene refers to the nucleotide sequences which encode the amino acids found in the nascent polypeptide as a result of translation of a mRNA molecule. The coding region is bounded, in eukaryotes, on the 5'side by the nucleotide triplet "ATG" which encodes the initiator methionine and on the 3'-side by one of the three triplets which specify stop codons (i.e., TAA, TAG, TGA). In addition to containing introns, genomic forms of a gene may also include sequences located on both the 5'- and 3'-end of the sequences which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers which control or influence the transcription of the gene. The 3'-flanking region may contain sequences which direct the termination of transcription, posttranscriptional cleavage and polyadenylation.

The terms "polypeptide", "peptide", "oligopeptide", "polypeptide", "gene product", "expression product" and "protein" are used interchangeably herein to refer to a polymer or oligomer of consecutive amino acid residues.

The term "isolated" as used herein means that a material has been removed from its original environment. For example, a naturally-occurring polynucleotide or polypeptide present in a living animal is not isolated, but the same polynucleotide or polypeptide, separated from some or all of the coexisting materials in the natural system, is isolated. Such polynucleotides can be part of a vector and/or such polynucleotides or polypeptides could be part of a composition, and would be isolated in that such a vector or composition is not part of its original environment.

The term "wild-type", "natural" or of "natural origin" means with respect to an organism, polypeptide, or nucleic acid sequence, that said organism is naturally occurring or available in at least one naturally occurring organism which is not changed, mutated, or otherwise manipulated by man.

The term "transgenic" or "recombinant" as used herein (e.g., with regard to a wheat zygote or wheat plants) is intended to refer to cells and/or plants that have incorporated exogenous genes or DNA sequences, including but not limited to genes or DNA sequences which are perhaps not normally present, genes not normally transcribed and translated ("expressed") in a given cell type, or any other genes or DNA sequences which one desires to introduce into the non-transformed cell and/or plant, such as genes which may normally be present in the non-transformed cell and/or plant but which one desires to have altered expression.

Preferably, the terms "transgenic" or "recombinant" with respect to, for example, a nucleic acid sequence (or an organism, expression cassette or vector comprising said nucleic acid sequence) refers to all those constructs originating by recombinant methods in which either
a) said nucleic acid sequence, or
b) a genetic control sequence linked operably to said nucleic acid sequence a), for example a promoter, or
c) (a) and (b)
is not located in its natural genetic environment or has been modified by recombinant methods, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the nucleic acid sequence is preferably retained, at least in part. The environment flanks the nucleic acid sequence at least at one side and has a sequence of at least 50 bp, preferably at least 500 bp, especially preferably at least 1000 bp, very especially preferably at least 5000 bp, in length. A naturally occurring expression cassette - for example the naturally occurring combination of a promoter with the corresponding gene - becomes a recombinant expression cassette when it is modified by non-natural, synthetic "artificial" methods such as, for example, mutagenization. Such methods have been described (US 5,565,350; WO 00/15815). Preferably, the term "recombinant" with respect to nucleic acids as used herein means that the nucleic acid is covalently joined and adjacent to a nucleic acid to which it is not adjacent in its natural environment. "Recombinant" polypeptides or proteins refer to polypeptides or proteins produced by recombinant DNA techniques, i. e., produced from cells transformed by an exogenous recombinant DNA construct encoding the desired polypeptide or protein. Recombinant nucleic acids and polypeptide may also comprise molecules which as such does not exist in nature but are modified, changed, mutated or otherwise manipulated by man.

A "recombinant polypeptide" is a non-naturally occurring polypeptide that differs in sequence from a naturally occurring polypeptide by at least one amino acid residue. Preferred methods for producing said recombinant polypeptide and/or nucleic acid may comprise directed or non-directed mutagenesis, DNA shuffling or other methods of recursive recombination.

The terms "heterologous nucleic acid sequence" or "heterologous DNA" are used interchangeably to refer to a nucleotide sequence which is ligated to a nucleic acid sequence to which it is not ligated in nature, or to which it is ligated at a different location in nature. Heterologous DNA is not endogenous to the cell into which it is introduced, but has been obtained from another cell. Generally, although not necessarily, such heterologous DNA encodes RNA and proteins that are not normally produced by the cell into which it is expressed.

The "efficiency of transformation" or "frequency of transformation" as used herein can be measured by the number of transformed cells (or transgenic organisms grown from individual transformed cells) that are recovered under standard experimental conditions (i.e. standardized or normalized with respect to amount of cells contacted with foreign DNA, amount of delivered DNA, type and conditions of DNA delivery, general culture conditions etc.) For example, when isolated zygotes are used as starting material for transformation, the frequency of transformation can be expressed as the number of transgenic plant lines obtained per 100 isolated zygotes transformed.

The term "cell" refers to a single cell. The term "cells" refers to a population of cells. The population may be a pure population comprising one cell type. Likewise, the population may comprise more than one cell type. In the present invention, there is no limit on the number of cell types that a cell population may comprise. The cells may be synchronize or not synchronized, preferably the cells are synchronized.

The term "chromosomal DNA" or "chromosomal DNA-sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like e.g., PCR analysis, Southern blot analysis, fluorescence in situ hybridization (FISH), and in situ PCR.

An "embryo sac" is typically an eight-nucleate female gametophyte. The embryo sac arises from the megaspore by successive mitotic divisions.

A "megaspore" is one of the four haploid spores originating from the meiotic division of the diploid megaspore mother cell in the ovary and which gives rise to the mega-gametophyte.

A "microspore" is one of the four haploid spores originating from the meiotic division of the diploid microspore mother cell in the anther and which gives rise to the pollen grain.

The "polar nuclei" are the two nuclei of the central cell which occupies the central region of the embryo sac. Upon fusion of the central cell with one of the sperm cells, the polar nuclei unite with the sperm nucleus. The fertilized central cell constitutes the cellular origin of the endosperm.

The term "protoplasts" means protoplasts originating from any plant tissues where their entire wall is removed. They may be isolated from leaves, seeds, flowers, roots, pollen, embryos, ovaries or ovules or the protoplasts may be isolated from cell cultures including immature pollen, immature pollen derived embryos, zygotic embroys, secondary embryos from zygotic or immature pollen-derived embryos or immature pollen-derived cell-cultures. It is common knowledge how to isolate protoplasts by enzymatic removal of the cell wall. Techniques for this are described (in e.g. Potrykus 1986; Kranz et al. 1991 ).

The term "structural gene" as used herein is intended to mean a DNA sequence that is transcribed into mRNA which is then translated into a sequence of amino acids characteristic of a specific polypeptide.

The term "expression" refers to the biosynthesis of a gene product. For example, in the case of a structural gene, expression involves transcription of the structural gene into mRNA and - optionally - the subsequent translation of mRNA into one or more polypeptides.

The term "transformation" includes introduction of genetic material into plant cells, preferably resulting in chromosomal integration and stable heritability through meiosis. Transformation also includes introduction of genetic material into plant cells in the form of plant viral vectors involving epichromosomal replication and gene expression which may exhibit variable properties with respect to meiotic stability.

The term "expression cassette" or "expression construct" as used herein is intended to mean the combination of any nucleic acid sequence to be expressed in operable linkage with a promoter sequence and - optionally - additional elements (like e.g., terminator and/or polyadenylation sequences) which facilitate expression of said nucleic acid sequence.

The term "promoter" as used herein is intended to mean a DNA sequence that directs the transcription of a DNA sequence (e.g., a structural gene). Typically, a promoter is located in the 5' region of a gene, proximal to the transcriptional start site of a structural gene. If a promoter is an inducible promoter, then the rate of transcription increases in response to an inducing agent. In contrast, the rate of transcription is not regulated by an inducing agent if the promoter is a constitutive promoter. Also, the promoter may be regulated in a tissue-specific or tissue preferred manner such that it is only active in transcribing the associated coding region in a specific tissue type(s) such as leaves, roots or meristem.

The term "operable linkage" or "operably linked" is to be understood as meaning, for example, the sequential arrangement of a regulatory element (e.g. a promoter) with a nucleic acid sequence to be expressed and, if appropriate, further regulatory elements (such as e.g., a terminator) in such a way that each of the regulatory elements can fulfill its intended function to allow, modify, facilitate or otherwise influence expression of said nucleic acid sequence. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those in which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. Operable linkage, and an expression cassette, can be generated by means of customary recombination and cloning techniques as described (e.g., in Maniatis 1989; Silhavy 1984; Ausubel 1987; Gelvin 1990). However, further sequences which, for example, act as a linker with specific cleavage sites for restriction enzymes, or as a signal peptide, may also be positioned between the two sequences. The insertion of sequences may also lead to the expression of fusion proteins. Preferably, the expression cassette, consisting of a linkage of promoter and nucleic acid sequence to be expressed, can exist in a vector-integrated form and be inserted into a plant genome, for example by transformation.

The term "androgenetically developing pollen" is intended to mean any deviation from the designated development of a microspore into a functional pollen grain. In contrast to the normal pollen formation, androgenetically developing pollen undergoes a number of successive cell divisions to form non-embryogenic or embroygenic callus or an embryo-like structure. These structures which genetically correspond to the male gametophyte can develop into haploid plants. The development of these structures may also include spontaneous or artificially induced genome doubling which may result in regeneration of homozygous, fertile plants.

### DETAILED DESCRIPTION OF THE INVENTION

The method according to the invention is remarkable in that the transformed zygotes are able without additional treatment by e.g., hormones to regenerate into green fertile plants. Furthermore it is not necessary to introduce a selectable gene. The present invention has the advantage of being genotype-independent. Since zygote development is a univeral step in higher plants and in particular within the Gramineae family, the methods disclosed herein can be transferred at least to other members of said family.

The terms "Gramineae" or "Graminaceae" as used herein intents to comprise all plants species of the Gramineae (Poaceae) family, especially those employed as foodstuffs or feeding stuffs such as rice, maize, wheat or other cereal species such as barley, millet and sorghum, rye, triticale or oats, and sugar cane, and all grass species. Furthermore included are the mature plants, seed, shoots and seedlings, and parts, propagation material and cultures derived therefrom, for example cell cultures. Mature plants refers to plants at any developmental stage beyond that of the seedling. The term seedling refers to a young immature plant in an early developmental stage, at which it is still dependent upon assimilates stored within the seed (e.g. in the endosperm, perisperm or cotyledons.

Included are all genera of the subfamilies Bambusoideae (e.g., the genus bamboo), Andropogonoideae (e.g., the genera Saccharum, Sorghum, or Zea), Arundineae (e.g., the genus Phragmites), Oryzoideae (e.g., the genus Oryza), Panicoideae (e.g., the genera Panicum, Pennisetum, and Setaria), Pooideae (Festuciadeae) (e.g., the genera Poa, Festuca, Lolium, Trisetum, Agrostis, Phleum, Dactylis, Alopecurus, Avena, Triticum, Secale, and Hordeum).

Preferred are Avena sativa (oats), Bambusa sp. and Bambusa bambos (bamboo), Saccharum officinarum (sugar cane), Triticum dicoccum (Emmer wheat), Triticum monococcum (Einkorn wheat), Triticum spelta (spelt wheat), Triticum durum (wheat), Triticum turgidum, Triticum aestivum (wheat), Zea mays (maize/corn), Panicum mili-aceum (common millet), Pennisetum thiphoides (Bulrush millet), Hordeum vulgare or H. sativum (barley), Oryza sativa (rice), Zizania aquatica (wild rice), Secale cereale (rye), Sorghum bicolor (S. vulgare) (sorghum). More preferred are wheat species and species with nearly identical zygote development characteristics like barley or maize. Especially preferred are all wheat species especially of the Triticum family (including both winter and spring wheat), more especially Triticum spp.: common (T. aestivum), durum (T. durum), spelt (T. spelta), Triticum dicoccum (Emmer wheat), Triticum turgidum, and Triticum monococcum (Einkorn wheat), with T. aestivum being particularly preferred. The method of the invention can be used to produce transgenic plants from spring wheats, such as, for example, Bobwhite, Marshall, PIVOT1, UC702, and Panewawa as well as from winter wheats, such as, for example, HY368, Neeley, FL302, RH91, R332, R1269 and R585. However, it should be pointed out, that the method of the invention is not limited to certain varities but is highly genotype-independent.

### ZYGOTE ISOLATION

Isolated zygoes as used within the method of the invention have demonstrated a high potential for regeneration.

The term "isolated zygote" as used herein means the fertilzed egg cell of a Gramineae plant substantially free of the surrounding tissue (like, e.g. pericarp, the two integuments, nucellus cells, embryo sac, constituting cells of the embryo sac).

The zygote in higher plants is per definition totipotent, i.e., its natural fate (function) is to develop into an intact plant.

The ovule is located inside the ovary and consists of the two integuments, nucellus tissue and the embryo sac. The embryo sac consists of a large central cell with two central or polar nuclei. At the chalazal end a number of antipodal cells are present while the two synergids and the egg cell are located within the micropylar region of the embryo sac. The structure of the embryo sac before, during and after fertilization has been described (Mogensen 1982; Engel 1989). In barley and wheat, fertilization occurs within about one hour after pollination: The pollen tube grows through the stigmata towards the ovule, then along the integuments, and eventually enters the micropyle and one of the synergids, which at this time has degenerated. There, the two sperm cells are released from the pollen tube, and one fuses with the egg while the other one fuses with the central cell. The fertilized, triploid central cell will at a later stage give rise to the triploid endosperm. Within about one day, wheat and barley zygotes have completed their first division which results in the two-celled proembryo.

Different methods for the isolation of Gramineae zygotes have been described (Holm et al. 1994, Leduc et. al. 1996, Kumlehn et al. 1998; see also Example 1). The unfertilized egg cell is a true protoplast. However, since immediately after fertilization cell wall formation starts, the zygote is by definition not a protoplast. However, may features of a protoplast still exist.

The behavior of the zygote is to some extent depending on the osmotic pressure of the medium. The osmotic pressure of the medium thus has to be of an order of magnitude where the zygote neither is swelling to an extent that it ruptures or undergoes extensive shriveling due to dehydration. Osmotic pressures ranging from 250 to 750 mOsm/kg can be tolerated, in particular a pressure of 375 mOsm/kg is suitable for barley zygote isolation, and an osmolality of about 600 mOsm/kg for wheat zygote isolation. The osmotic pressure can be increased or reduced by addition/removal of e.g. mannitol. For example, a 0.55 M mannitol solution guarantees an appropriate osmotic pressure for wheat zygote isolation. During isolation, the zygotes may become sticky in the presence of exogenously supplied Ca²⁺ making the cells very difficult to handle. Therefore, in a preferred embodiment pure mannitol solution is used in which the isolated zygotes were morphologically stable and did not show any obvious loss of viability during preparation.

With regard to wheat, efficient and reproducible embryo development has been obtained from fertilized wheat *(Triticum aestivum* L.) egg cells isolated 3-6 h after hand-pollination of emasculated spikes. It is possible to routinely isolate viable zygotes from about 75% of the processed pistils from cultivars of both winter and spring type.

### AGROBACTERIUM TRANSFORMATION

The genetic component is introduced into the isolated zygote by means of Agrobacterium-mediated DNA transfer.

The term "Agrobacterium" as used herein means all species of the Agrobacterium family (including Agrobacterium tumefaciens and Agrobacterium rhizogenes). Preferably, transformation is realized utilizing strains of Agrobacterium tumefaciens or Agrobacterium rhizogenes. The principles of plant transformation by means of Agrobacterium-mediated DNA transfer are well known in the art (see above; BACKGROUND OF THE INVENTION; Horsch 1985).

Agrobacterium tumefaciens and A. rhizogenes are plant-pathogenic soil bacteria, which genetically transform plant cells. The Ti and Ri plasmids of A. tumefaciens and A. rhizogenes, respectively, carry genes responsible for genetic transformation of the plant (Kado 1991). Vectors are based on the Agrobacterium Ti- or Ri-plasmid and utilize a natural system of DNA transfer into the plant genome. As part of this highly developed parasitism Agrobacterium transfers a defined part of its genomic information (the T-DNA; flanked by about 25 bp repeats, named left and right border) into the chromosomal DNA of the plant cell (Zupan 2000). By combined action of the so called vir genes (part of the original Ti-plasmids) said DNA-transfer is mediated. For utilization of this natural system, Ti-plasmids were developed which lack the original tumor inducing genes ("disarmed vectors"). In a further improvement, the so called "binary vector systems", the T-DNA was physically separated from the other functional elements of the Ti-plasmid (e.g., the vir genes), by being incorporated into a shuttle vector, which allowed easier handling (EP-A 120 516; US 4.940.838). These binary vectors comprise (beside the disarmed T-DNA with its border sequences), prokaryotic sequences for replication both in Agrobacterium and E. coli. It is an advantage of Agrobacterium-mediated transformation that in general only the DNA flanked by the borders is transferred into the genome and that preferentially only one copy is inserted. Descriptions of Agrobacterium vector systems and methods for Agrobacterium-mediated gene transfer are known in the art (Miki 1993; Gruber 1993; Moloney 1989).

Hence, for Agrobacteria-mediated transformation the genetic composition (e.g., comprising an expression cassette) is integrated into specific plasmids, either into a shuttle or intermediate vector, or into a binary vector. If a Ti or Ri plasmid is to be used for the transformation, at least the right border, but in most cases the right and left border, of the Ti or Ri plasmid T-DNA is linked to the expression cassette to be introduced in the form of a flanking region. Binary vectors are preferably used. Binary vectors are capable of replication both in E.coli and in Agrobacterium. They may comprise a selection marker gene and a linker or polylinker (for insertion of e.g. the expression cassette to be transferred) flanked by the right and left T-DNA border sequence. They can be transferred directly into Agrobacterium (Holsters 1978). The selection marker gene permits the selection of transformed Agrobacteria and is, for example, the nptll gene, which confers resistance to kanamycin. The Agrobacterium which acts as host organism in this case should already contain a plasmid with the vir region. The latter is required for transferring the T-DNA to the plant cell. An Agrobacterium transformed in this way can be used for transforming plant cells. The use of T-DNA for transforming plant cells has been studied and described intensively (EP 120 516; Hoekema 1985; An et al. 1985).

Common binary vectors are based on "broad host range"-plasmids like pRK252 (Bevan 1984) or pTJS75 (Watson et al. 1985) derived from the P-type plasmid RK2. Most of these vetors are derivatives of pBIN19 (Bevan 1984). Various binary vectors are known, some of which are commercially available such as, for example, pBI101.2 or pBIN19 (Clontech Laboratories, Inc. USA). Additional vectors were improved with regard to size and handling (e.g. pPZP; Hajdukiewicz 1994). Improved vector systems are described also in WO 02/00900.

In a preferred embodiment, Agrobacterium strains for use in the practice of the invention include octopine strains, e.g., LBA4404 or agropine strains, e.g., EHA101 or EHA105. Suitable strains of A. tumefaciens for DNA transfer are for example EHA101 pEHA101 (Hood et al. 1986), EHA105[pEHA105] (Li 1992), LBA4404[pAL4404] (Hoekema 1983), C58C1[pMP90] (Koncz & Schell 1986), and C58C1[pGV2260] (Deblaere 1985). Other suitable strains are Agrobacterium tumefaciens C58, a nopaline strain. Other suitable strains are A. tumefaciens C58C1 (Van Larebeke 1974), A136 (Watson 1975) or LBA4011 (Klapwijk 1980). In a preferred embodiment, the Agrobacterium strain used to transform the plant tissue pre-cultured with the plant phenolic compound contains a L,L-succinamopine type Ti-plasmid, preferably disarmed, such as pEHA101. In another preferred embodiment, the Agrobacterium strain used to transform the plant tissue pre-cultured with the plant phenolic compound contains an octopine-type Ti-plasmid, preferably disarmed, such as pAL4404. Generally, when using octopine-type Ti-plasmids or helper plasmids, it is preferred that the virF gene be deleted or inactivated (Jarschow 1991).

The method of the invention can also be used in combination with particular Agrobacterium strains, to further increase the transformation efficiency, such as Agrobacterium strains wherein the vir gene expression and/or induction thereof is altered due to the presence of mutant or chimeric virA or virG genes (e.g. Hansen 1994; Chen and Winans 1991; Scheeren-Groot et al., 1994).

Preferred are further combinations of Agrobacterium tumefaciens strain LBA4404 (Hiei 1994) with super-virulent plasmids. These are preferably pTOK246-based vectors (Ishida 1996).

A binary vector or any other vector can be modified by common DNA recombination techniques, multiplied in E. coli, and introduced into Agrobacterium by e.g., electroporation or other transformation techniques (Mozo 1991).

Agrobacterium is grown and used in a manner similar to that described in Ishida (Ishida et al. 1996). The vector comprising Agrobacterium strain may, for example, be grown for 3 days on YP medium (5 g/l yeast extract, 10 g/l peptone, 5 g/l Nail, 15 g/l agar, pH 6.8) supplemented with the appropriate antibiotic (e.g., 50 mg/l spectinomycin). Bacteria are collected with a loop from the solid medium and resuspended. In a preferred embodyment of the invention, Agrobacterium cultures are started by use of aliquots frozen at -80°C. For Agrobacterium treatment of isolated zygotes, the bacteria are resuspended in the medium used for zygote culture.

The zygote to be transformed may be treated with cell wall degrading enzymes to facilitate or enhance transformation efficacy. However, especially for wheat zygotes isolated within 20 hours after pollination this degradation step is not necessary and is omitted in a preferred embodiment of the invention. The concentration of Agrobacterium used for infection and co-cultivation may need to be varied. It is understood in the art that high densities and long co-cultivation can injure the isolated zygotes. Thus, a range of Agrobacterium concentrations from 10⁵ - 10¹⁰ cfu/ml and a range of co-cultivation periods from a few hours to 7 days are tested with the isolated zygotes. The co-cultivation of Agrobacterium with the isolated zygotes is in general carried out for 1 to five, preferably 2 to 3 days. In an improved embodiment of the invention the isolated zygotes may be treated with a phenolic compound prior to or during the Agrobacterium co-cultivation. "Plant phenolic compounds" or "plant phenolics" suitable within the scope of the invention are those isolated substituted phenolic molecules which are capable to induce a positive chemotactic response, particularly those who are capable to induce increased vir gene expression in a Ti-plasmid containing Agrobacterium sp., particularly a Ti-plasmid containing Agrobacterium tumefaciens. Methods to measure chemotactic responses towards plant phenolic compounds have been like e.g., described (Ashby 1988) and methods to measure induction of vir gene expression are also well known (Stachel 1985; Bolton 1986). The pre-treatment and/or treatment during Agrobacterium co-cultivation has at least two beneficial effects: Induction of the vir genes of Ti plasmids or helper plasmids (Van Wordragen 1992; Jacq 1993; James 1993; Guivarc'h 1993), and enhancement of the competence for incorporation of foreign DNA into the genome of the plant cell.

Preferred plant phenolic compounds are those found in wound exudates of plant cells. One of the best known plant phenolic compounds is acetosyringone, which is present in a number of wounded and intact cells of various plants, albeit in different concentrations. However, acetosyringone (3,5-dimethoxy-4-hydroxyacetophenone) is not the only plant phenolic which can induce the expression of vir genes. Other examples are α-hydroxy-acetosyringone, sinapinic acid (3,5-dimethoxy-4-hydroxycinnamic acid), syringic acid (4-hydroxy-3,5 dimethoxybenzoic acid), ferulic acid (4-hydroxy-3-methoxycinnamic acid), catechol (1,2-dihydroxybenzene), p-hydroxybenzoic acid (4-hydroxybenzoic acid), β-resorcylic acid (2,4-dihydroxybenzoic acid), protocatechuic acid (3,4-dihydroxybenzoic acid), pyrrogallic acid (2,3,4-trihydroxybenzoic acid), gallic acid (3,4,5-trihydroxybenzoic acid) and vanillin (3-methoxy-4-hydroxybenzaldehyde), and these phenolic compounds are known or expected to be able to replace acetosyringone in the cultivation media with similar results. As used herein, the mentioned molecules are referred to as plant phenolic compounds.

Plant phenolic compounds can be added to the plant culture medium either alone or in combination with other plant phenolic compounds. A particularly preferred combination of plant phenolic compounds comprises at least acetosyringone and p-hydroxybenzoic acid, but it is expected that other combinations of two, or more, plant phenolic compounds will also act synergistically in enhancing the transformation efficiency.

Moreover, certain compounds, such as osmoprotectants (e.g. L-proline preferably at a concentration of about 700 mg/L or betaine), phytohormes (inter alia NAA), opines, or sugars, are expected to act synergistically when added in combination with plant phenolic compounds.

In one embodiment of the invention, it is preferred that the plant phenolic compound, particularly acetosyringone is added to the medium prior to contacting the isolated zygotes with Agrobacteria (for e.g., several hours to one day). The exact period in which the cultured cells are incubated in the medium containing the plant phenolic compound such as acetosyringone, is believed not to be critical and only limited by the time the zygote starts entering mitosis.

The concentration of the plant phenolic compound in the medium is also believed to have an effect on the development of competence for integrative transformation. The optimal concentration range of plant phenolic compounds in the medium may vary depending on the species from which the zygote derived, but it is expected that about 100 µM to 700 µM is a suitable concentration for many purposes. However, concentrations as low as approximately 25 µM can be used to obtain a good effect on transformation efficiency. Likewise, it is expected that higher concentrations up to approximately 1000 µM will yield similar effects. Comparable concentrations apply to other plant phenolic compounds, and optimal concentrations can be established easily by experimentation in accordance with this invention.

Agrobacteria to be co-cultivated with the isolated zygotes can be either pre-incubated with acetosyringone or another plant phenolic compound, as known by the person skilled in the art, or used directly after isolation from their culture medium. Particularly suited induction conditions for Agrobacterium tumefaciens have been described by Vernade et al. (1988). Efficiency of transformation with Agrobacterium can be enhanced by numerous other methods known in the art like for example vacuum infiltration (WO 00/58484), heat shock and/or centrifugation, addition of silver nitrate, sonication etc.

Supplementation of the co-culture medium with antioxidants (e.g., dithiothreitol), phenol-absorbing compounds (like polyvinylpyrrolidone, Perl et al. 1996) or thiol compounds (e.g., L-cysteine, Olhoft et al. 2001) which can decrease tissue necrosis due to plant defence responses (like phenolic oxidation) may further improve the efficiency of Agrobacterium-mediated transformation.

It has been observed within this invention that transformation efficacy of the isolated zygotes by Agrobacterium can be significantly improved by keeping the pH of the co-cultivation medium in a range from 5.4 to 6.4, preferably 5.6 to 6.2, especially preferably 5.8 to 6.0. In an improved embodiment of the invention stabilisation pf the pH in this range is mediated by a combination of MES and potassium hydrogenphosphate buffers.

### CULTIVATION OF ZYGOTES AND REGENERATION INTO PLANTS

After transformation, the zygote is transferred into another Millicell insert placed in the same petri dish and the co-culture insert is removed to avoid an ongoing impact of the Agrobacteria on the medium (nutrient depletion, release of bacterial exudates, modification of pH). It turned out to be crucial to keep the isolated zygotes or zygote-derived structures within the same medium, because even a minor shift in concentration of some media components or in osmolality causes subsequent degeneration of the cultured cells. Since the medium used for co-culture is basically suitable for embryo development, only some supplementation appeared to be necessary, e.g. antibiotics (like e.g., Amoxicillin, Cefotaxime, Clavulanic acid, Timentin or a combination thereof) and additional CaCl₂ to stop the development of the Agrobacteria as well as additional glutamine to support embryonic development.

In a preferred embodiment of the invention, the in vitro development of the zygotes and their subsequent regeneration into fertile plants is mediated by co-cultivation techniques. The co-cultivation principle is well established in tissue culture, where e.g., protoplasts often are co-cultivated with suspension cultures to support protoplast development (Evans 1983). There is today very little evidence on the nature of the factors which during the co-cultivation support regeneration and embryogenesis. Methods for co-cultivation to be preferably utilized herein are described (e.g., in WO 94/01999; Kumlehn et al. 1998).

Several kinds of cells and/or tissues are suitable for regeneration of the zygotes into embryos. Within this invention, these cells and/or tissues are subsumed under the term "feeder system". As feeder systems, embryogenic as well as non-embryogenic suspensions can be used. Preferably, a co-cultivation with isolated immature pollen (preferably barley pollen) or isolated immature pollen which had been stimulated to androgenetic development is used. Alternatively, cultures of wheat pistils (gynoecia) can be employed for feeder systems. Methods to co-culture ovaries are known in the art (Köhler and Wenzel 1978, Mejza et al. 1993). Furthermore, a combination of the above mentioned feeder systems can be used like e.g., a combination of barley immature pollen culture with wheat pistil culture.

Co-culture with e.g. barley pollen cultures resulted in embryonic development of the cultivated wheat zygotes. Within 23 h of pollination the zygotes underwent the first cell division and then formed differentiated embryos very similar to those grown in planta.

Pollen embryogenesis is one of the few tissue culture systems available where a very large number of cells simultaneously undergo embryonic development. Immature barley pollen with their high regeneration potential seem to condition the medium with a complex combination of compounds which support embryonic development of zygotes within the method of the invention.

This complex mixture of components may be continuously released when immature pollen undergoes embryogenesis. As an alternative to the co-cultivation procedure, it is thus possible, to use conditioned medium or extracts of immature pollen or anthers, in particular pollen, which is undergoing embryogenesis. Extracts are defined as non-intact cellular materials, which are isolated from pollen or anthers, f.ex. by blending and possibly further purified by filtration or fractionation.

In general, immature pollen or anthers from any species or variety may be used but the regeneration frequency of protoplasts, cells and tissues may depend on the source of the feeder tissue. The regeneration frequency also depends on the cell density of the pollen culture or the anthers cultivated in the medium. Immature pollen or anthers from barley (e.g., the barley cultivar Igri) are particularly suitable for use in the method according to the invention. In a preferred embodiment, immature pollen of cv. Igri are isolated and cultured as basically described by Mordhorst and Lörz (1993) and transferred to the medium used for zygote culture as an intermediate step.

It is preferred to use immature barley pollen cultures at a density between 10² to 10⁶, preferably 10³ to 3*10⁵, especially preferably 10⁴ and 10⁵ immature pollen per ml of culture medium or the equivalent amount of anthers. It is preferred, however, to use immature pollen. Suspension cultures or callus cultures derived from immature pollen can also be used (Roberts-Oehlschlager et al. 1990). It is preferred though to use young cultures of immature pollen or anthers, i.e., preferably cultures that are younger than 30 days, and in particular younger than 21 days, more preferably cultures between 5 and 14 days.

Feeder system-mediated co-cultivation may involve a physical separation between the supporting feeder system and the isolated zygotes to be supported and where it is ensured that low and high molecular compounds can diffuse between the two cultures

The androgenetically developing pollen and the plant material to be regenerated can be cultured in the same medium which preferably is liquid or semi-liquid or the pollen-derived microcalli can be cultivated in one medium and the plant material to be regenerated in a second medium which is in exchangeable contact with the first medium or contain fractions of the first medium. This can, for example be realized by cultivating the isolated zygotes in cell culture dishes with bottom semi-permeable membranes (e.g. Millicell inserts; Millipore, Bedford, Mass., USA) placed in larger cell culture dishes comprising the pollen culture.

A co-cultivation system may of course be constructed in a number of ways. The immature pollen may be cultured directly in the petri dish thereby avoiding the use of a large insert. The pollen-derived microcalli and the isolated zygotes to be regenerated may also be present in the same reservoir or dish, thereby avoiding the use of any of the two inserts. The isolated zygotes to be regenerated may be embedded in a solid medium such as agarose which is permeable for the components present in the pollen culture. The only essential principle in the co-cultivation is thus that the components present in the pollen culture undergoing embryogenesis readily can reach the material (e.g., the zygotes) to be regenerated.

It was found that the number of zygotes, cultivated per Millicell insert did not influence the embryonic development that is, even a singly cultivated zygote can develop into a well-differentiated embryo (Kumlehn et al. 1998).

It is of course obvious that the growth and culture media used for the tissue culture contain the nutrients essential for the plant material. The composition of nutrients in such growth and culture media are common knowledge to any person experienced in the field (e.g., Kao 1975; Linsmaier 1965; Spencer 1990; Twell 1989a). The N6Z medium has demonstrated to be especially preferably for promoting embryogenesis from isolated wheat zygotes (Table 1).

The feeder system can be employed only after the Agrobacterium co-cultivation step but may be also - preferably - employed immediately after isolation of the zygote and remained during the Agrobacterium co-cultivation. In another preferred embodiment of the invention, the Agrobacteria are kept separate from the feeder system and only contact with the zygotes to be transformed is allowed. This may be ensured by incubating the zygotes with the Agrobacteria within a Millicell insert having a pore width of 0.4 µm which prevents diffusion of Agrobacteria into the medium outside this insert as well as contamination of the feeder system. The feeder system may be cultured directly in a larger cell culture plate comprising the Millicell used for co-culture of the zygotes with Agrobacteria (Fig. 1). This approach is advantageous since contamination of the feeder system with Agrobacterium is prevented. This allows to use the very same feeder system also after removal of the Agrobacterium for further incubation/regeneration of the zygotes. Zygotes would be simply transferred to another Millicell insert and again cultivated within the before used feeder system comprising plate (supplemented with antibiotics and/or Calcium chloride to prevent growth of remaining Agrobacteria in the zygote culture). This variant is especially preferred in another improved embodiment of the invention, since it helps to overcome certain difficulties related to sensitivity of zygotes to changed environmental conditions (like e.g., changed medium composition, osmolality and pH).

In a preferred embodiment, a co-culture with barley immature pollen which had been stimulated to androgenetic development is employed. This resulted in embryonic development of the cultivated wheat zygotes with high efficiency. Within 23 h of pollination, the zygotes underwent their first cell division. They proceeded to develop into club-shaped embryos, most of which turned subsequently to dorsoventral differentiation. The morphological patterns of in-vitro-grown embryos were in accordance with those of normal zygotic embryos growing in planta. Optimized media are disclosed in examples 4 and 5 (Table 1). More than 80% of the zygotes eventually developed into plants (e.g., in genotypes Florida and Veery #5, respectively). All regenerated plants were morphologically normal and fertile.

After development of zygote-derived embryos (preferably of a size of at least 1 mm; which is obtained for example after three to four weeks of co-cultivation with immature pollen in the dark at 25°C), these embryos may further cultivated towards development of plantlets and plants. For this purpose numerous methods known to the person skilled in the art may be employed. For example, the zygote-derived embryos may be transferred (plated) e.g., on solidified medium (e.g., K4NT, Table 1) supporting further embryo differentiation, germination and plantlet formation. The medium used in this step can be any medium which permits germination of embryos. In one embodiment, a shoot-producing compound is added to the medium. These shoot-producing compounds are well known in the art (Mursahige and Skoog, 1962; Kasha 1990). Such compounds include plant growth regulators and include IAA, IBA, and BA at low concentrations (Becker er al., 1994; Vasil 1992). In another embodiment of the invention, a medium free of a plant growth regulator can be used to induce shoot formation (Weeks 1993). Subculture can be carried out on a monthly basis until plantlets are transferred to soil.

If necessary, explants with shoots can be transferred to medium particularly supporting root formation. These media are well known in the art (Weeks 1993; Vasil 1992). One preferred root-producing medium is a modified MS-medium without any plant growth regulator (Murashige 1962; Zhou 1992). Once roots have been formed, the plants can be transferred to soil and grown following methods known in the art to produce mature plants with seeds.

In another embodiment of the invention, the media as employed during the method of the invention may be optionally further supplemented with one or more plant growth regulator, like e.g., cytokinin compounds (e.g., 6-Benzylaminopurin) or auxin compounds (e.g., 2,4-D). The term "plant growth regulator" (PGR) as used herein means naturally occurring or synthetic (not-naturally occurring) compounds that can regulate plant growth and development. PGRs may act singly or in consort with one another or with other compounds (e.g., sugars, amino acids).

The term "auxin" or "auxin compounds" comprises compounds which stimulate cellular elongation and division, differentiation of vascular tissue, fruit development, formation of adventitious roots, production of ethylene, and - in high concentrations - induce dedifferentiation (callus formation). The most common naturally occurring auxin is indoleacetic acid (IAA), which is transported polarly in roots and stems. Synthetic auxins are used extensively in modern agriculture. Auxin compounds comprise indole-3-butyric acid (IBA), naphthylacetic acid (NAA), and 2,4-dichlorphenoxyacetic acid (2,4-D), wherein 2,4-D is a more active compound.

The term "cytokinin" or "cytokinin compound" comprises compounds which stimulate cellular division, expansion of cotyledons, and growth of lateral buds. They delay senescence of detached leaves and, in combination with auxines (e.g. IAA), may influence formation of roots and shoots. Cytokinin compounds comprise, for example, 6-isopentenyladenine (IPA) and 6-benzyladenine/6-benzylaminopurine (BAP).

In a preferred embodiment the method of the invention is carried out under conditions which does not lead to dedifferentiation. Preferably, no dedifferentiation is induced to the isolated zygote and zygote-derived embryo, plantlet or plant. More preferably, no embryonic tissue dedifferentiation and/or formation of callus or callus-like structures is initiated.

The term "dedifferentiation" as used herein is intended to mean the process of formation of rapidly dividing cells without particular function in the scope of the plant body.

These cells often possess an increased potency with regard to its ability to develop into various plant tissues. Preferably the term is intended to mean the reversion of a differentiated or specialized tissues to a more pluripotent or totipotent (e.g., embryonic) form. Dedifferentiation may lead to reprogramming of a plant tissue (revert first to undifferentiated, non-specialized cells. then to new and different paths). The term "totipotency" as used herein is intended to mean a plant cell containing all the genetic and/or cellular information required to form an entire plant. Dedifferentiation can be initiated by certain plant growth regulators (e.g., auxin and/or cytokinin compounds), especially by certain combinations and/or concentrations thereof.

In a preferred embodiment, the concentration and/or combination of auxins and/or cyt-kinin added to a culture medium used within the method of the invention is kept in a range, where no dedifferentiation (e.g., callus induction) is initiated. Combinations of auxins/cytokinins leading to shoot and/or root induction may be advantageously employed during plant regeneration from the zygote-derived embryo.

Within an preferred embodiment of the method of the invention cytokinins and auxins may be utilized to induce shoot and/or root development, but not dedifferentiation. However, under certain circumstances, dedifferentiation of a transformed zygotic embryo in a later step of the method may be advantageous (e.g., to increase the probability of systemically (non-chimeric) transformation of the Gramineae plant obtained by the method of the invention or to multiply certain transformation events). In general, however, preferably no dedifferentiation is applied.

More preferably no auxin compound (like e.g., 2,4-D) are added or their concentration is kept in a low range of 0 to about 0.2 mg/l 2,4-D.

The media as used herein (e.g., for cultivation of zygotes or zygote-derived embryos) may be liquid media (e.g., for pollen and zygote cultures) or solid media (e.g., solidified by 2-7 g/l Gelrite® (Sigma Chemical, St. Louis, MO) or 4 g/l low-melting agarose).

Other important aspects of the invention include the progeny of the transgenic plants prepared by the disclosed methods, as well as the cells derived from such progeny, and the seeds obtained from such progeny.

Within this invention a highly efficient transformation and regeneration protocol for isolated Gramineae (especially wheat) zygotes undergoing direct embryo differentiation when co-cultured with immature pollen. Almost every wheat zygote can undergo embryonic development with morphological patterns homologous to normal zygotic embryos growing in planta. In this way, it is possible to regenerate plant materials, which previously have been difficult to regenerate from cell cultures into green fertile plants in much higher frequencies than seen before and it is also possible to regenerate plant materials, which previously have been impossible to regenerate.

### SELECTION

The method of the invention reliably leads to transgenic, non-chimeric gramineae plants with high efficiency. Therefore, utilization of selectable or screenable marker genes for identification of the transgenic plants is not necessary. Because of the reservations of the public with regard to antibiotic and other selectable markers this is considered to be an economically highly relevant advantage over the methods known in the art.

The insertion of the genetic component into the chromosomal DNA can be demonstrated and analyzed by various methods known in the art like e.g., PCR analysis, Southern blot analysis, fluorescence in situ hybridization (FISH), and in situ PCR.

However, insertion of a selectable and/or screenable marker is comprised within the scope of the method of the invention. This may be advantageous e.g., for later use as a herbicide-resistance trait. Therefore, in another embodiment of the invention the genetic component introduced into the genome of the Gramineae plant comprises an expression cassette able to express a selectable or screenable marker in said Gramineae plant. The medium employed for culturing the transformed zygotes and/or zygote-derived embryos and/or plantlets may further contain a compound used to select zygotes, embryos or plantlets containing a selectable or screenable DNA sequence.

The compound used for said selection or screening can be any of a variety of well known selection compounds, such as antibiotics and herbicides. Preferred compounds can include geneticin (G-418) (aminoglycoside) (Nehra et al. 1994), glyphosate (Della-Cioppa et al. 1987) and bialaphos (Vasil et al. 1992; Weeks et al. 1993). The availability of alternative selection agents is an important requirement for commercial application of agriculture biotechnology. The use of kanamycin has been less successful for cereal crops because of the high endogenous level of tolerance (Dekeyser et al. 1989). Bialaphos has been widely used as a selection agent in cereal crop transformation (Weeks et al. 1993; Vasil et al. 1993; Becker et al. 1994; Nehra et al. 1994; Wan and Lemaux 1994). Further preferred selectable and screenable marker genes are disclosed below.

Transformed plant material (e.g., cells, embryos, tissues or plantlets) which express such marker genes are capable of developing in the presence of concentrations of a corresponding selection compound (e.g., antibiotic or herbicide) which suppresses growth of an untransformed wild type tissue. The resulting plants can be bred and hybridized in the customary fashion. Two or more generations should be grown in order to ensure that the genomic integration is stable and hereditary. Corresponding methods are described, (Jenes 1993; Potrykus 1991).

### PREFERRED GENETIC COMPONENTS

Preferably, the genetic component (e.g., the T-DNA) inserted into the genome of the target plant comprises at least one expression cassette, which may - for example - facilitate expression of selection markers, trait genes, antisense RNA or double-stranded RNA. Preferably said expression cassettes comprise a promoter sequence functional in plant cells operatively linked to a nucleic acid sequence which - upon expression - confers an advantageous phenotype to the so transformed plant. The person skilled in the art is aware of numerous sequences which may be utilized in this context, e.g. to increase quality of food and feed, to produce chemicals, fine chemicals or pharmaceuticals (e.g., vitamins, oils, carbohydrates; Dunwell 2000), conferring resistance to herbicides, or conferring male sterility. Furthermore, growth, yield, and resistance against abiotic and biotic stress factors (like e.g., fungi, viruses or insects) may be enhanced. Advantageous properties may be conferred either by overexpressing proteins or by decreasing expression of endogenous proteins by e.g., expressing a corresponding antisense (Sheehy 1988; US 4,801,340; Mol 1990) or double-stranded RNA (Matzke 2000; Fire 1998; Waterhouse 1998; WO 99/32619; WO 99/53050; WO 00/68374; WO 00/44914; WO 00/44895; WO 00/49035; WO 00/63364).

For expression in plants, plant-specific promoters are preferred. The term "plant-specific promoter" is understood as meaning, in principle, any promoter which is capable of governing the expression of genes, in particular foreign genes, in plants or plant parts, plant cells, plant tissues or plant cultures. In this context, expression can be, for example, constitutive, inducible or development-dependent. The following are preferred:

### a) Constitutive promoters

"Constitutive" promoters refers to those promoters which ensure expression in a large number of, preferably all, tissues over a substantial period of plant development, preferably at all times during plant development. A plant promoter or promoter originating from a plant virus is especially preferably used. The promoter of the CaMV (cauliflower mosaic virus) 35S transcript (Franck 1980; Odell 1985; Shewmaker 1985; Gardner 1986) or the 19S CaMV promoter (US 5,352,605; WO 84/02913; Benfey 1989) are especially preferred. Another suitable constitutive promoter is the rice actin promoter (McElroy 1990), Rubisco small subunit (SSU) promoter (US 4,962,028), the legumin B promoter (GenBank Acc. No. X03677), the promoter of the nopalin synthase from Agrobacterium, the TR dual promoter, the OCS (octopine synthase) promoter from Agrobacterium, the ubiquitin promoter (Holtorf et al. 1995), the ubiquitin 1 promoter (Christensen et al. 1989, 1992; Bruce et al. 1989), the Smas promoter, the cinnamyl alcohol dehydrogenase promoter (US 5,683,439), the promoters of the vacuolar AT-Pase subunits, the pEMU promoter (Last et al. 1991); the MAS promoter (Velten et al. 1984) and maize H3 histone promoter (Lepetit et al. 1992; Atanassova et al. 1992), the promoter of the Arabidopsis thaliana nitrilase-1 gene (GenBank Acc. No.: U38846, nucleotides 3862 to 5325 or else 5342) or the promoter of a proline-rich protein from wheat (WO 91/13991), and further promoters of genes whose constitutive expression in plants, especially in monocot or Gramineae plants is known to the skilled worker. The maize ubiquitin promoter is particularly preferred in wheat and barley.

### b) Tissue-specific or tissue-preferred promoters

Furthermore preferred are promoters with specificities for seeds, such as, for example, the phaseolin promoter (US 5,504,200; Bustos et al. 1989, Murai et al.1983; Sengupta-Gopalan et al. 1985), the promoter of the 2S albumin gene (Joseffson et al. 1987), the legumine promoter (Shirsat 1989), the USP (unknown seed protein) promoter (Bäumlein 1991a), the napin gene promoter (US 5,608,152; Stalberg et al. 1996), the promoter of the sucrose binding proteins (WO 00/26388) or the legumin B4 promoter (LeB4; Bäumlein et al. 1991b, 1992), the Arabidopsis oleosin promoter (WO 98/45461), and the Brassica Bce4 promoter (WO 91/13980). Promoters which are furthermore preferred are those which permit a seed-specific expression in monocots such as maize, barley, wheat, rye, rice and the like. The promoter of the Ipt2 or Ipt1 gene (WO 95/15389, WO 95/23230) or the promoters described in WO 99/16890 (promoters of the hordein gene, the glutelin gene, the oryzin gene, the prolamin gene, the gliadin gene, the glutelin gene, the zein gene, the casirin gene or the secalin gene) can advantageously be employed. Further preferred are a leaf-specific and light-induced promoter such as that from cab or rubisco (Simpson 1985; Timko 1985); an anther-specific promoter such as that from LAT52 (Twell et al. 1989b); a pollen-specific promoter such as that from Zml3 (Guerrero et al. 1993); and a microspore-preferred promoter such as that from apg (Twell et al. 1993).

### c) Chemically inducible promoters

The expression cassettes may also contain a chemically inducible promoter (review article: Gatz et al. 1997), by means of which the expression of the exogenous gene in the plant can be controlled at a particular point in time. Such promoters such as, for example, the PRP1 promoter (Ward et al. 1993), a salicylic acid-inducible promoter (WO 95/19443), a benzenesulfonamide-inducible promoter (EP 0 388 186), a tetracyclin-inducible promoter (Gatz et al. 1991, 1992), an abscisic acid-inducible promoter EP 0 335 528) or an ethanol-cyclohexanone-inducible promoter (WO 93/21334) can likewise be used. Also suitable is the promoter of the glutathione-S transferase isoform II gene (GST-II-27), which can be activated by exogenously applied safeners such as, for example, N,N-diallyl-2,2-dichloroacetamide (WO 93/01294) and which is operable in a large number of tissues of both monocots and dicots. Further exemplary inducible promoters that can be utilized in the instant invention include that from the ACE1 system which responds to copper (Mett et al. 1993); or the In2 promoter from maize which responds to benzenesulfonamide herbicide safeners (Hershey et al. 1991; Gatz et al. 1994). A promoter that responds to an inducing agent to which plants do not normally respond can be utilized. An exemplary inducible promoter is the inducible promoter from a steroid hormone gene, the transcriptional activity of which is induced by a glucocorticosteroid hormone (Schena et al. 1991).

Particularly preferred are constitutive promoters. Furthermore, further promoters may be linked operably to the nucleic acid sequence to be expressed, which promoters make possible the expression in further plant tissues or in other organisms, such as, for example, E.coli bacteria. Suitable plant promoters are, in principle, all of the above-described promoters.

The genetic component and/or the expression cassette may comprise further genetic control sequences in addition to a promoter. The term "genetic control sequences" is to be understood in the broad sense and refers to all those sequences which have an effect on the materialization or the function of the expression cassette according to the invention. For example, genetic control sequences modify the transcription and translation in prokaryotic or eukaryotic organisms. Preferably, the expression cassettes according to the invention encompass a promoter functional in plants 5'-upstream of the nucleic acid sequence in question to be expressed recombinantly, and 3'-downstream a terminator sequence as additional genetic control sequence and, if appropriate, further customary regulatory elements, in each case linked operably to the nucleic acid sequence to be expressed recombinantly.

Genetic control sequences furthermore also encompass the 5'-untranslated regions, introns or noncoding 3'-region of genes, such as, for example, the actin-1 intron, or the Adh1-S introns 1, 2 and 6 (general reference: The Maize Handbook, Chapter 116, Freeling and Walbot, Eds., Springer, New York (1994)). It has been demonstrated that they may play a significant role in the regulation of gene expression. Thus, it has been demonstrated that 5'-untranslated sequences can enhance the transient expression of heterologous genes. Examples of translation enhancers which may be mentioned are the tobacco mosaic virus 5' leader sequence (Gallie et al. 1987) and the like. Furthermore, they may promote tissue specificity (Rouster et al. 1998).

The expression cassette may advantageously comprise one or more enhancer sequences, linked operably to the promoter, which make possible an increased recombinant expression of the nucleic acid sequence. Additional advantageous sequences, such as further regulatory elements or terminators, may also be inserted at the 3' end of the nucleic acid sequences to be expressed recombinantly. Polyadenylation signals which are suitable as control sequences are plant polyadenylation signals, preferably those which essentially correspond to T-DNA polyadenylation signals from *Agrobacterium tumefaciens,* in particular the OCS (octopin synthase) terminator and the NOS (nopalin synthase) terminator.

Control sequences are furthermore to be understood as those permitting removal of the inserted sequences from the genome. Methods based on the cre/lox (Sauer B 1998; Odell 1990; Dale 1991), FLP/FRT (Lysnik 1993), or Ac/Ds system (Wader et al. 1987; US 5,225,341; Baker et al. 1987; Lawson et al. 1994) permit a - if appropriate tissue-specific and/or inducible - removal of a specific DNA sequence from the genome of the host organism. Control sequences may in this context mean the specific flanking sequences (e.g., lox sequences), which later allow removal (e.g., by means of cre recombinase).

The genetic component and/or expression cassette of the invention may comprise further functional elements. The term functional element is to be understood in the broad sense and refers to all those elements which have an effect on the generation, amplification or function of the genetic component, expression cassettes or recombinant organisms according to the invention. Functional elements may include for example (but shall not be limited to):
1. Selection markers
   Selection markers are useful to select and separate successfully transformed or homologous recombined cells.
   1.1 Positive selection markers
      Selection markers confer a resistance to a biocidal compound such as a metabolic inhibitor (e.g., 2-deoxyglucose-6-phosphate, WO 98/45456), antibiotics (e.g., kanamycin, G 418, bleomycin or hygromycin) or herbicides (e.g., phosphinothricin or glyphosate). Especially preferred selection markers are those which confer resistance to herbicides. Examples which may be mentioned are:
      - Phosphinothricin acetyltransferases (PAT; also named Bialophos ®resistance; bar; de Block 1987; . EP 0 333 033; US 4,975,374)
      - 5-enolpyruvylshikimate-3-phosphate synthase (EPSPS) conferring resistance to Glyphosate® (N-(phosphonomethyl)glycine) (Shah 1986)
      - Glyphosate® degrading enzymes (Glyphosate® oxidoreductase; gox),
      - Dalapon® inactivating dehalogenases (deh)
      - sulfonylurea- and imidazolinone-inactivating acetolactate synthases (for example mutated ALS variants with, for example, the S4 and/or Hra mutation
      - Bromoxynil® degrading nitrilases (bxn)
      - Kanamycin- or. G418- resistance genes (NPTII; NPTI) coding e.g., for neomycin phosphotransferases (Fraley 1983)
      - 2-Desoxyglucose-6-phosphate phosphatase (DOG^{R}1-Gene product; WO 98/45456; EP 0 807 836) conferring resistance against 2-desoxyglucose (Randez-Gil 1995).
      - hygromycin phosphotransferase (HPT), which mediates resistance to hygromycin (Vanden Elzen 1985).
      - dihydrofolate reductase (Eichholtz 1987)

      Additional positive selectable marker genes of bacterial origin that confer resistance to antibiotics include the aadA gene, which confers resistance to the antibiotic spectinomycin, gentamycin acetyl transferase, streptomycin phosphotransferase (SPT), aminoglycoside-3-adenyl transferase and the bleomycin resistance determinant (Hayford 1988; Jones 1987; Svab 1990; Hille 1986).
   1.2) Negative selection marker
      Negative selection markers are especially suitable to select organisms with defined deleted sequences comprising said marker (Koprek et al. 1999). Examples for negative selection marker comprise thymidin kinases (TK), cytosine deaminases (Gleave et al. 1999; Perera et al. 1993; Stougaard 1993), cytochrom P450 proteins (Koprek et al. 1999), haloalkan dehalogenases (Naested 1999), iaaH gene products (Sundaresan et al. 1995), cytosine deaminase codA (Schlaman & Hooykaas 1997), or tms2 gene products (Fedoroff & Smith 1993).
2) Reporter genes
   Reporter genes encode readily quantifiable proteins and, via their color or enzyme activity, make possible an assessment of the transformation efficacy, the site of expression or the time of expression. Very especially preferred in this context are genes encoding reporter proteins (Schenborn 1999) such as the green fluorescent protein (GFP) (Sheen 1995; Haseloff 1997; Reichel 1996; Tian 1997; WO 97/41228; Chui 1996; Leffel 1997), chloramphenicol transferase, a luciferase (Ow 1986; Millar 1992), the aequorin gene (Prasher 1985), β-galactosidase, R locus gene (encoding a protein which regulates the production of anthocyanin pigments (red coloring) in plant tissue and thus makes possible the direct analysis of the promoter activity without addition of further auxiliary substances or chromogenic substrates (Dellaporta 1988; Ludwig 1990), with β-glucuronidase (GUS) being very especially preferred (Jefferson 1987a,b). β-glucuronidase (GUS) expression is detected by a blue color on incubation of the tissue with 5-bromo-4-chloro-3-indolyl-β-D-glucuronic acid, bacterial luciferase (LUX) expression is detected by light emission; firefly luciferase (LUC) expression is detected by light emission after incubation with luciferin; and galactosidase expression is detected by a bright blue color after the tissue is stained with 5-bromo-4-chloro-3-indolyl-β-D-galactopyranoside. Reporter genes may also be used as scorable markers as alternatives to antibiotic resistance markers. Such markers are used to detect the presence or to measure the level of expression of the transferred gene. The use of scorable markers in plants to identify or tag genetically modified cells works well only when efficiency of modification of the cell is high.
3) Origins of replication, which ensure amplification of the expression cassettes or vectors according to the invention in, for example, E. coli. Examples which may be mentioned are ORI (origin of DNA replication), the pBR322 ori or the P15A ori (Maniatis 1989).
4) Elements which are necessary for Agrobacterium-mediated plant transformation, such as, for example, the right or left border of the T-DNA or the vir region.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the composition, methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims. All publications, patents, and patent applications cited herein are hereby incorporated by reference for all purposes.

### Sequences

1. SEQ ID NO: 1      Binary vector pUGAB7

2. SEQ ID NO: 2      Binary vector pSUNUbi-GFP

3. SEQ ID NO: 3      Binary vector pSUNUbiGUS

### Examples:

### General methods:

Unless otherwise specified, all chemicals were from Fluka (Buchs), Merck (Darmstadt), Roth (Karlsruhe), Serva (Heidelberg) and Sigma (Deisenhofen). Restriction enzymes, DNA-modifying enzymes and molecular biological kits were from Amersham-Pharmacia (Freiburg), Biometra (Göttingen), Roche (Mannheim), New England Biolabs (Schwalbach), Novagen (Madison, Wisconsin, USA), Perkin-Elmer (Weiterstadt), Qiagen (Hilden), Stratagen (Amsterdam, Netherlands), Invitrogen (Karlsruhe) and Ambion (Cambridgeshire, United Kingdom). The reagents used were employed in accordance with the manufacturer's instructions.

For example, oligonucleotides can be synthesized chemically in the known manner using the phosphoamidite method (Voet, 2nd edition, Wiley Press New York, pages 896-897). The cloning steps carried out for the purposes of the present invention such as, for example, restriction cleavages, agarose gel electrophoreses, purification of DNA fragments, transfer of nucleic acids to nitrocellulose and nylon membranes, linking DNA fragments, transformation of E. coli cells, bacterial cultures, multiplication of phages and sequence analysis of recombinant DNA, are carried out as decribed (Maniatis 1989). Recombinant DNA molecules were sequenced using an ABI laser fluorescence DNA sequencer following the method of Sanger (Sanger 1977).

Plant material: Culture of wheat *(Triticum aestivum* L.) plants, emasculation of spikes and hand-pollination were conducted as described (Kumlehn et al. 1997). As genotypes, the German winter-type cultivar Certo and the spring-type Bobwhite were used.

DNA-Constructs: The Agrobacterium binary constructs pSUNUbiGUS (SEQ ID NO: 3) and pSUNUbiGFP (SEQ ID NO: 2) for marker gene expression were generated using the pSUN300, a derivative of the pSUN binary vector (WO 02/00900). pSUNUbiGUS and pSUNUbiGFP were generated by insertion of a Ubi-1 promoter/ GUS(intron)/ 35S-T expression cassette and a Ubi-1 promoter/ GFP/ 35S-T expression cassette respectively into the binary vector. Thereafter the P-nos/ nptll/ nosT cassette of pSUN300 was replaced by a Ubi-1-promoter/pat/35S-T cassette to apply both binary plasmids to wheat transformation.

### Example 1: Isolation of wheat zygotes

Wheat spikes with emasculated florets of cvs. Certo or Bobwhite were harvested 1 to 5 hours after manual pollination. Upon removal of the outer glumes and the lemmas, the prepared spikes were surface-sterilized in 2% sodium hypochloride supplemented with 0.03 % Tween 20 for 10 minutes, washed 4 to 5 times with sterile water, and excess water was removed by use of sterile filter paper. By use of a standard preparation microscope, the basal tips of the excised pistils were cut off and immediately transferred to 0.55 M mannitol solution in a 35-mm petri dish. For efficient isolation of viable zygotes, it was necessary to cut off the ovary tips precisely. To avoid squashing of the ovary and its embryo sac this cut had to be carried out with a very sharp blade. From these explants, the tip of the inner integument which contained at least a part of the embryo sac was dissected out of the outer integument and the pericarp tissue which were then removed from the solution. Isolation of the zygote was more efficient when the outer integument was removed from the ovule tip during dissection. This way, the cells of the embryo sac can be recognized more easily during further processing of the tissue. Using an inverted microscope (Axiovert 200M, Carl Zeiss, Oberkochen/ Germany), the zygotes were released from the surrounding ovule tissue by means of fine-tipped glass needles. Since the wheat embryo sac is not as tightly embedded in nucellus tissue as, for instance, the maize embryo sac, the zygote can be isolated efficiently without use of cell-wall-degrading enzymes. With this technique, it was possible to routinely recover viable zygotes from about 75% of the excised pistils in genotypes of both winter and spring types. The isolated zygotes had a diameter of around 75 µm.

### Example 2: Barley immature pollen culture

Barley tillers of cv. Igri were harvested when the tips of the awns were just emerging from the sheeth of the flag leaf. The tillers were surface-sterilized by spraying with 70% ethanol. After having released a spike from the flag leaf, the awns were removed with forceps and five spikes prepared like this were put onto a 70-mm sterile filter paper disc which had been dipped once in sterile distilled water and placed into a 9-cm petri dish. The dishes were sealed and incubated at 4°C for about 4 weeks in the dark. To isolate immature pollen, 10 to 15 pretreated spikes were chopped down to 1 to 2 cm pieces to be transferred to a pre-cooled (4°C) Waring Blendor. The Blendor was filled with 20 ml of cold (4°C) 0.4 M mannitol and the spikes blended 2 times for 5 seconds at low speed. The solution was poured through a 100-µm nylon mesh into an autoclaved Magenta box. The debris was slightly squashed, rinsed with 5 ml of 0.4 M mannitol and transferred back into the blendor. Blending was repeated using 10 ml of 0.4 mannitol solution. The interior of the blendor was rinsed with 5 ml of mannitol solution which was subsequently poured through the remaining debris in the mesh. The pollen suspension was transferred from the Magenta box into a 50-ml polypropylene tube which was closed with a screw cap and centrifuged at 100 g for 10 min at 4°C. The supernatant was removed and the pellet resuspended in 5 ml of 0.55 M maltose. The suspension was equally distributed to two 12-ml centrifuge tubes and cautiously overlayered with 2 x 1.5 ml of 0.4 M mannitol. After gradient centrifugation at 100 g for 10 minutes, the interphase bands containing the vacuolated pollen were transferred to a 50-ml polypropylene tube which were filled up to 20 ml with 0.4 M mannitol. The population density was assessed by means of a haemacytometer and the vacuolated pollen centrifuged again at 100 g for 10 minutes. The supernatant was discarded and the population density was adjusted to 2.5 x 10⁵ per ml by adding KBP medium (Table 1). One-ml aliquots of the suspension were distributed to 35-mm petri dishes. The cultures were incubated at 25°C in the dark.

### Example 3: Growth of Agrobacterium tumefaciens to be used for zygote transformation

A cryostock of LBA4404/ pSB1/ pUGAB7 consisting of 200 µl of a log-phase *Agrobacterium* culture with a density of 10⁹ cfu per ml and 200 µl of 15 % sterile glycerol solution was taken from -80°C and added to 10 ml of CPY medium supplemented with 10 mg/l tetracyclin and 150 mg /l spectinomycin. The culture was incubated at 28°C on a rotary shaker at 180 rpm in the dark for about 24 hours. The Agrobacteria were pelleted at 4000 rpm for 10 minutes and the supernatant removed. The pellet was resuspended in fresh medium without antibiotics and incubated on a rotary shaker (∼60 rpm) at 25°C in the dark for 1 to 3 hours.

### Example 4: Co-culture of isolated wheat zygotes with immature barley pollen-derived microcalli and Agrobacterium tumefaciens

Two Millicell inserts of 12 mm diameter and 0.4 µm pore size (Millipore, Molsheim/France) were placed in a 35-mm petri dish (Greiner, Frickenhausen/Germany) with 1.5 ml of ZT1 medium (Table 1). One ml of ZT1 medium containing about 5000 androgenetically developing microcalli taken from a 2-week old barley pollen culture were added. The Millicell inserts were filled with 200 µl of ZT1 medium each. Upon isolation in 0.55 M mannitol, five to 10 wheat zygotes of cvs. Certo or Bobwhite were transferred in about 100 nl of 0.55 M mannitol solution by means of a glass capillary interfaced by teflon tube to a Cell Tram (Eppendorf, Hamburg/Germany) into one of the Millicell inserts. To ensure sufficient space for embryo formation, not more than ten zygotes were cultivated per insert. A 24-h culture of *Agrobacterium tumefaciens* strain LBA4404 carrying the binary vector pUGAB7 was adjusted to a population density of 10⁹ cfu per ml by adding ZT1 medium accordingly. Two µl of the resulting suspension was transferred to the Millicell insert that contained the isolated zygotes to give a final *Agrobacterium* density of 10⁷ cfu per ml. The co-culture dishes were incubated on a rotary shaker (~60 rpm) at 21°C in the dark for 48 hours.

### Example 5: Embryonic development and transgenic plant regeneration from isolated wheat zygotes co-cultivated with Agrobacterium tumefaciens

After the co-culture period, the zygotes were transferred by means of the above mentioned capillary system in about 100 nl of medium to the second Millicell insert placed within the same 35-mm petri dish. The Millicell insert which had been used for co-culture was removed. Upon transfer of the zygotes or zygote-derived structures, the ZT1-medium inside the petri dish was supplemented with 13.5 µl of a Timentin (Duchefa, Netherlands) stock solution (40 mg/ml) and 18.9 µl of a 1 M CaCl₂ stock solution in order to stop the development of the Agrobacteria. Furtheron, 32.4 µl of a 0.25 M glutamine solution was added to the medium to support embryonic development. The sealed dishes were incubated on a rotary shaker (~60 rpm) at 25°C in the dark for 7 days and then further cultured without shaking under the same conditions. Zygote-derived embryos of at least 1 mm size were transferred to K4NT medium (Table 1) for regeneration. Genomic DNA from regenerated plantlets was isolated and analysed for integration of the transgenes by standard methods, i.e. PCR and Southern blot.

**Table 1**

| Components | | | | |
|---|---|---|---|---|
| [mg·l⁻¹] | N6Z | KBP | ZT1 | K4NT |
| NH₄NO₃ | - | 80 | - | 320 |
| (NH₄)₂SO₄ | 231 | - | 231 | - |
| KNO₃ | 1,415 | 2,022 | 1,415 | 3,640 |
| KH₂PO₄ | 200 | 340 | 1,360 | 340 |
| CaCl₂·2H₂O | 83 | 441 | 441 | 441 |
| MgSO₄·7H₂O | 93 | 246 | 93 | 246 |
| NaFeEDTA | 25 | 27.5 | 25 | 27.5 |
| MnSO₄·4H₂O | 4.0 | 11.2 | 4.0 | 11.2 |
| H₃BO₃ | 0.5 | 3.1 | 0.5 | 3.1 |
| ZnSO₄·7H₂O | 0.5 | 7.2 | 0.5 | 7.2 |
| Na₂MoO₄·2H₂O | 0.025 | 0.12 | 0.025 | 0.12 |
| CuSO₄·5H₂O | 0.025 | 0.025 | 0.025 | 1.25 |
| CoCl₂·6H₂O | 0.025 | 0.024 | 0.025 | 0.024 |
| Kl | - | 0.17 | - | 0.17 |
| retinol | 0.01 | 0.01 | 0.01 | - |
| thiamine·HCl | 1.0 | 1.0 | 1.0 | 10.0 |
| nicotinic acid | 1.0 | 1.0 | 1.0 | 1.0 |
| riboflavin | 0.2 | 0.2 | 0.2 | - |
| Ca-pantothenate | 1.0 | 1.0 | 1.0 | - |
| folic acid | 0.4 | 0.4 | 0.4 | - |
| pyridoxine·HCl | 1.0 | 1.0 | 1.0 | 1.0 |
| cobalamine | 0.02 | 0.02 | 0.02 | - |
| ascorbic acid | 2.0 | 2.0 | 2.0 | - |
| calciferol | 0.01 | 0.01 | 0.01 | - |
| biotin | 0.01 | 0.01 | 0.01 | - |
| cholin chloride | 1.0 | 1.0 | 1.0 | - |
| p-aminobenzoic acid | 0.02 | 0.02 | 0.02 | - |
| myo-inositol | 100 | 100 | 100 | - |
| malic acid | 400 | - | - | - |
| citric acid | 40 | - | - | - |
| fumaric acid | 40 | - | - | - |
| Na-pyruvate | 20 | | - | - |
| glutamine | 1,000 | 438.4 | 438.4 | 146 |
| casein hydrolysate | 250 | - | - | - |
| myo-inositol | 100 | - | 100 | 100 |
| xylose | 150 | - | 150 | - |
| glucose | 85,000 | - | 90,000 | - |
| maltose·H₂O | - | 90,000 | - | 36,000 |
| 2,4-D | 0.2 | - | - | - |
| BAP | - | 0.22 | - | 0.22 |
| MES | - | - | 1,950 | - |
| Acetosyringone | - | - | 100 | - |
| Phytagel | - | - | - | 4,000 |
| pH | 5.7 | 5.8 | 5.9 | 5.8 |

### REFERENCES

The references listed below and all references cited herein are incorporated herein by reference to the extent that they supplement, explain, provide a background for, or teach methodology, techniques, and/or compositions employed herein.
- 1.: Allington "Micromanipulation of the Unfixed Cereal Embryo Sac," in THE EXPERIMENTAL MANIPULATION OF OVULE TISSUES 39-51 (Longman, New York, 1985)
- 2.: An et al. (1985) EMBO J 4:277-287
- 3.: Ashby et al. (1988) J. Bacteriol. 170: 4181-4187
- 4.: Atanassova et al. (1992) Plant J 2(3): 291-300
- 5.: Ausubel FM et al. (1987) Current Protocols in Molecular Biology, Greene Publishing Assoc. and Wiley Interscience
- 6.: Baker et al. (1987) EMBO J 6: 1547-1554
- 7.: Bäumlein et al. (1992) Plant J 2(2):233-9;
- 8.: Bäumlein H et al. (1991a) Mol Gen Genet 225(3):459-467
- 9.: Bäumlein H et al. (1991b) Mol Gen Genet 225:121-128
- 10.: Becker et al. (1992) Plant Mol. Biol. 20: 49
- 11.: Backer et al. (1994) Plant J., 5:299-307,
- 12.: Benfey et al.(1989) EMBO J 8:2195-2202
- 13.: Bevan et al. (1984) Nucl Acid Res 12,8711-8720
- 14.: Bolton et al. (1986) Science 232: 983-985;
- 15.: Bruce et al. (1989) Proc Natl Acad Sci USA 86:9692-9696
- 16.: Bustos MM et al. (1989) Plant Cell 1(9):839-53)
- 17.: Chen and Winans (1991) J. Bacteriol. 173: 1139-1144
- 18.: Christensen et al. (1989) Plant Mol. Biol. 12: 619-632
- 19.: Christensen et al. (1992) Plant Mol Biol 18:675-689
- 20.: Christou et al. (1988) Plant Physiol 87:671-674
- 21.: Christou et al. (1991) Bio/Technology 9:957-962
- 22.: Chu CC et al. (1990) Plant Sci 66:255- 767
- 23.: Chui WL et al. (1996) Curr Biol 6:325-330;
- 24.: Creissen et al. (1991) Plant J. 2: 129
- 25.: Dale & Ow (1991) Proc Nat'l Acad Sci USA 88:10558-10562
- 26.: Datta et al. (1990b) Bio/Technology 8:736-740
- 27.: Datta SK & Wenzel G (1987) Plant Sci 48:49-54
- 28.: Datta SK et al. (1990a) Plant Sci. 67, 83-88
- 29.: Davey et al. (1991) J Exp Bot 42:1129-1169
- 30.: de Block et al. (1987) EMBO J 6:2513-2518
- 31.: De la Pena et al. (1987) Nature 325:274-276
- 32.: Deblaere et al. (1985) Nucl Acids Res 13:4777-4788
- 33.: Dekeyser et al. (1989) Plant Physiol 90:217-223
- 34.: Della-Cioppa et al. (1987) Bio/Technology 5:579-584
- 35.: Dellaporta et al. (1988) In: Chromosome Structure and Function: Impact of New Concepts, 18th Stadler Genetics Symposium, 11:263-282
- 36.: Du et al. (1989) Genet Manip Plants 5:8-12
- 37.: Dunwell JM (2000) J Exp Bot 51 Spec No:487-96
- 38.: Eichholtz et al. (1987) Somatic Cell and Molecular Genetics 13, 67-76
- 39.: Engell K (1989). Nord. J. Bot. 9, 265-280.
- 40.: EP-A1 0 120 516
- 41.: EP-A1 0 120 516
- 42.: EP-A1 0 333 033
- 43.: EP-A1 0 335 528
- 44.: EP-A1 0 388 186
- 45.: EP-A1 0 807 836
- 46.: Evans DA & Bravo JE (1983). Protoplast isolation and culture. In: D.A. Evans, W.R. Sharp, Ammirato and Y. Yamada eds. Handbook of Plant Cell Culture. Macmillan Publishing Company, V. 1, 124-176.
- 47.: Fedoroff NV & Smith DL (1993) Plant J 3:273- 289
- 48.: Fire A. et al (1998) Nature 391:806-811
- 49.: Fontes et al. (1991) Plant Cell 3:483-496
- 50.: Fraley et al. (1983) Proc Natl Acad Sci USA 80:4803
- 51.: Franck et al. (1980) Cell 21:285-294
- 52.: Fromm et al. (1986) Nature 319:791-793
- 53.: Fromm et al. (1990) Bio/Technology 8:833-839
- 54.: Gallie et al. (1987) Nucl Acids Res 15:8693-8711
- 55.: Gardner et al. (1986) Plant Mol Biol 6:221-228
- 56.: Gatz et al. (1991) Mol Gen Genetics 227:229-237
- 57.: Gatz et al. (1992) Plant J 2:397-404
- 58.: Gatz et al. (1994) Mol Gen Genetics 243:32-38
- 59.: Gatz et al. (1997) Annu Rev Plant Physiol Plant Mol Biol 48:89-108
- 60.: Gelvin et al. (Eds) (1990) Plant Molecular Biology Manual; Kluwer Academic Publisher, Dordrecht, The Netherlands
- 61.: GenBank Acc. No. X03677
- 62.: GenBank Acc. No.: U38846, nucleotides 3862 to 5325 or else 5342
- 63.: Gleave AP et al. (1999) Plant Mol Biol. 40(2):223-35
- 64.: Gordon-Kamm et al. (1990) Plant Cell 2:603-618
- 65.: Gould et al. (1989) J Cell Biol 108:1657
- 66.: Gould et at. (1991) Plant Physiol 95(2):426-434
- 67.: Gruber et al. (1993) "Vectors for Plant Transformation," in METHODS IN PLANT MOLECULAR BIOLOGY AND BIOTECHNOLOGY; pp.89-119.
- 68.: Guerrero et al. (1993) Mol Gen Genet 224:161-168
- 69.: Guivarc'h et al. (1993) Protoplasma 174: 10-18
- 70.: Hajdukiewicz et al. (1994) Plant Mol Biol 25:989-994
- 71.: Hansen et al. (1994) Proc. Natl. Acad. Sci. USA 91:7603-7607
- 72.: Haseloff et al.(1997) Proc Natl Acad Sci USA 94(6):2122-2127;
- 73.: Hayakawa et al. (1992) Proc Natl Acad Sci USA 89:9865-9869
- 74.: Hershey et al. (1991) Mol Gen Genetics 227:229-237
- 75.: Hiei et al. (1994) Plant J 6: 271-282
- 76.: Hoekema (1985) In: The Binary Plant Vector System, Offsetdrukkerij Kanters B.V., Alblasserdam, Chapter V
- 77.: Hoekema et al. (1983) Nature 303:179-181
- 78.: Holm PB et al. (1994) Plant Cell 6: 531-543
- 79.: Holsters et al. (1978) Mol Gen Genet 163:181-187
- 80.: Holtorf S et al. (1995) Plant Mol Biol 29:637-649
- 81.: Hood et al. (1986) J Bacteriol 168:1291-1301
- 82.: Horsch RB et al. (1985) Science 225: 1229f
- 83.: Ishida Y et al. (1996) Nature Biotech 745-750
- 84.: Jacq et al. (1993) Plant Cell Reports 12: 621-624
- 85.: James et al. (1993) Plant Cell Reports 12: 559-563)
- 86.: Jarchow et al. (1991), Proc. Natl. Acad. Sci. USA 88:10426-10430
- 87.: Jefferson (1987b) Plant Mol. Bio. Rep., 5:387-405
- 88.: Jefferson et al. (1987a) EMBO J., 6:3901-3907
- 89.: Jenes B et al.(1993) Techniques for Gene Transfer, in: Recombinant Plants, Vol. 1, Engineering and Utilization, edited by SD Kung and R Wu, Academic Press, pp. 128-143
- 90.: Johri BM et al. ( 1992) Comparative embryology of angiosperms. Springer, Berlin
- 91.: Joseffson LG et al. (1987) J Biol Chem 262:12196-12201
- 92.: Kado (1991) Crit Rev Plant Sci 10:1
- 93.: Kao KN & Michayluk MR (1975) Planta (Berl.) 126, 105-110.
- 94.: Kasha et al. (1990) Gene Manip. Plant Improv. II, 213-239
- 95.: Klapwijk et al. (1980) J. Bacteriol., 141,128-136
- 96.: Knox et al. (1987) Plant Mol. Biol. 9:3-17
- 97.: Köhler and Wenzel (1978) J. Plant Physiol. 121: 181-191
- 98.: Koncz & Schell (1986) Mol Gen Genet 204:383-396
- 99.: Koop HU, Schweiger HG (1995) J Plant Physioi 111: 235-757
- 100.: Koprek T et al. (1999) Plant J 19(6): 719-726
- 101.: Kranz E et al. (1991) Ses Plant Reprod 4: 12-16
- 102.: Kranz E, Lörz H (1993) Plant Cell 5: 739-746
- 103.: Kranz E, Lörz H (1994) Zygote 2: 175-13s
- 104.: Kumlehn et al. (1997) Plant J. 12: 1473-1479
- 105.: Kumlehn J et al. (1998) Planta 205:327-333)
- 106.: Last DI et al. (1991) Theor. Appl. Genet. 81, 581-588.
- 107.: Lawson et al. (1994) Mol Gen Genet 245:608-615
- 108.: Leduc et al., Sex Plant Reprod 8:313-317 (1995)
- 109.: Leduc N et al. (1996) Dev Biol 177: 190-203
- 110.: Leffel SM et al. (1997) Biotechniques. 23(5):912-8
- 111.: Lepetit et al., Mol. Gen. Genet. 231: 276-285 (1992)
- 112.: Lerner et al., Plant Physiol. 91: 124-129 (1989)
- 113.: Li et al. (1992) Plant Mol Biol 20:1037-1048
- 114.: Linsmaier EM and Skoog F (1965) Physiol. Plant. 18, 100-127.
- 115.: Ludwig et al. (1990) Science 247:449
- 116.: Luo and Wu, PlantMol. Biol. Rep., 6:165-174, 1988.
- 117.: Lysnik et al. (1993) NAR 21:969-975
- 118.: Maniatis T, Fritsch EF and Sambrook J (1989) Molecular Cloning: A Laboratory Manual, 2nd Ed., Cold Spring Harbor Laboratory, Cold Spring Harbor (NY)
- 119.: Matsuoka et al., Proc. Nat'l Acad. Sci. 88: 834 (1991)
- 120.: Matzke MA et al. (2000) Plant Mol Biol 43:401-415
- 121.: McElroy et al., Plant Cell 2: 163171 (1990)
- 122.: Meinke DW (1993) Plant Cell 3: 857-866
- 123.: Mejza et al. (1993) Plant Cell Reports 12: 149-153
- 124.: Mett et al. PNAS 90: 4567-4571 (1993)
- 125.: Miki et al. (1993) "Procedures for Introducing Foreign DNA into Plants" in METHODS IN PLANT MOLECULAR BIOLOGY AND BIOTECHNOLOGY; pp.67-88
- 126.: Millar et al. (1992) Plant Mol Biol Rep 10:324-414
- 127.: Mogensen HL (1982) Carlsberg Res. Commun. 47, 313-354.
- 128.: Mol JN et al. (1990) FEBS Lett 268(2):427-430
- 129.: Moloney et al., Plant Cell Reports 8: 238 (1989)
- 130.: Mooney and Doodwin, Plant Cell Tissue Organ Culture, 25:209-218, 1991.
- 131.: Mordhorst AP, Lorz H (1993) J Plant Physiol 131: 485-491
- 132.: Mozo & Hooykaas, Plant Mol. Biol. 16 (1991), 917-918.
- 133.: Murai et al., Science 23: 476-482 (1983)
- 134.: Murashige T and Skoog F (1962) Physiol. Plant. 15, 472-497. (472/473)
- 135.: Naested H (1999) Plant J 18:571-576
- 136.: Nehra et al., Plant J., 5:285-297, 1994.
- 137.: Odell et al. (1985) Nature 313:810-812
- 138.: Odell et al. (1990) Mol Gen Genet 223:369-378
- 139.: Ow et al. (1986) Science 234:856-859;
- 140.: Paszkowski et al. (1984) EMBO J 3:2717-2722
- 141.: Perera RJ et al. (1993) Plant Mol. Biol 23(4): 793-799;
- 142.: Potrykus & Shillito RD (1986) Methods Enzymol. 118, 549-578.
- 143.: Potrykus (1990) Bio/technology. 8, 535-542.
- 144.: Potrykus (1991) Ann Rev Plant Physiol Plant Mol Biol 42:205-225
- 145.: Prasher et al. (1985) Biochem Biophys Res Commun 126(3):1259-1268)
- 146.: Raineri et al. (1990) Bio/Technology 8:33
- 147.: Randez-Gil et al. (1995) Yeast 11:1233-1240
- 148.: Rasco-Gaunt et al. (2001) J. Exp. Bot. 52: 865-874
- 149.: Reichel et al.(1996) Proc Natl Acad Sci USA 93(12):5888-5893;
- 150.: Rhodes CA et al. (1988). Science 240, 204-207.
- 151.: Rouster J et al. (1998) Plant J 15:435-440
- 152.: Sanford JC (1990) Physiologia Plantarium 79:206-209
- 153.: Sanger et al. (1977) Proc Natl Acad Sci USA 74:5463-5467
- 154.: Sauer B (1998) Methods 14(4):381-92
- 155.: Sautter et al. (1991) Bio/Technology, 9:1080-1085
- 156.: Scheeren-Groot et al. (1994) J. Bacteriol 176: 6418-6426
- 157.: Schena et al. (1991) Proc Nat'l Acad Sci USA 88:10421
- 158.: Schenborn E, Groskreutz D. (1999) Mol Biotechnol 13(1):29-44
- 159.: Schlaman HRM and Hooykaas PJJ (1997) Plant J 11:1377-1385
- 160.: Sengupta-Gopalan et al., Proc. Nat'l Acad. Sci. USA 82: 3320-3324 (1985)
- 161.: Shah et al. (1986) Science 233: 478
- 162.: Sheehy et al. (1988) Proc Natl Acad Sci USA 85: 8805-8809;
- 163.: Sheen et al.(1995) Plant J 8(5):777-784;
- 164.: Shewmaker et al. (1985) Virology 140:281-288
- 165.: Shillito et al. (1985) Bio/Technology, 3:1099-1103
- 166.: Shimamoto et al. (1989) Nature 338:274-276
- 167.: Shirsat A et al. (1989) Mol Gen Genet 215(2):326-331
- 168.: Silhavy TJ, Berman ML and Enquist LW (1984) Experiments with Gene Fusions, Cold Spring Harbor Laboratory, Cold Spring Harbor (NY)
- 169.: Simpson et al. (1985) EMBO J 4:2723-2729
- 170.: Somers et al. (1992) Bio/Technology 10:1589-1594
- 171.: Spencer TM et al. (1990) Theor Appl Genet 79, 625-631
- 172.: Stachel et al. (1985) Nature 318: 624-629
- 173.: Stalberg K et al. (1996) Planta 199:515-519
- 174.: Stougaard J; (1993) Plant J 3:755-761
- 175.: The Maize Handbook, Chapter 116, Freeling and Walbot, Eds., Springer, New York (1994)
- 176.: Theunis et al. (1991) Sex Plant Reprod. 4:145-154
- 177.: Thomas TL (1993) Plant Cell 5:1401-1410
- 178.: Tian et al. (1997) Plant Cell Rep 16:267-271;
- 179.: Timko et al. (1985) Nature 318: 579-582
- 180.: Topfer et al. (1989) Plant Cell, 1:133-139
- 181.: Twell et al. (1983) Sex. Plant Reprod. 6: 217-224
- 182.: Twell et al. (1989a) Plant Physiol 91:1270-1274
- 183.: Twell et al. (1989b) Mol Gen Genet 217:240-245
- 184.: US 4,801,340;
- 185.: US 4,962,028
- 186.: US 4,975,374)
- 187.: US 4.940.838
- 188.: US 5,225,341
- 189.: US 5,352,605
- 190.: US 5,504,200
- 191.: US 5,565,350
- 192.: US 5,608,152;
- 193.: US 5,683,439
- 194.: Van Laerebeke et al. (1974) Nature 252,169-170
- 195.: Van Wordragen and Dons (1992) Plant Mol. Biol. Rep. 10: 12-36
- 196.: Vanden Elzen et al. (1985) Plant Mol Biol. 5:299
- 197.: Vasil et al. (1992) Bio/Technology, 10:667-674
- 198.: Vasil et al. (1993) Bio/Technology, 11:1153-1158
- 199.: Velten J et al. (1984) EMBO J. 3(12): 2723-2730.
- 200.: Vernade et al. (1988) J. Bacteriol. 170: 5822-5829
- 201.: Wader et al., in TOMATO TECHNOLOGY 189-198 (Alan R. Liss, Inc. 1987)
- 202.: Wan & Lemaux (1994) Plant Physiol., 104:3748
- 203.: Ward et al. (1993) Plant Mol Biol 22:361-366
- 204.: Waterhouse PM et al. (1998) Proc Natl Acad Sci USA 95:13959-64
- 205.: Watson et al. (1975) J. Bacteriol 123, 255-264
- 206.: Watson et al. (1985) EMBO J 4(2):277- 284
- 207.: Weeks et al. (1993) Plant Physiol 102:1077-1084
- 208.: WO 00/15815
- 209.: WO 00/26388
- 210.: WO 02/00900
- 211.: WO 84/02913
- 212.: WO 91/02071
- 213.: WO 91/13980
- 214.: WO 91/13991
- 215.: WO 92/06205
- 216.: WO 93/01294
- 217.: WO 93/18168
- 218.: WO 93/21334
- 219.: WO 94/00583
- 220.: WO 94/00977
- 221.: WO 94/01999
- 222.: WO 94/48814
- 223.: WO 95/06722
- 224.: WO 95/15389
- 225.: WO 95/19443
- 226.: WO 95/23230
- 227.: WO 98/01576
- 228.: WO 98/45456
- 229.: WO 98/45461
- 230.: WO 99/16890
- 231.: WO 00/44895;
- 232.: WO 00/44914;
- 233.: WO 00/49035;
- 234.: WO 00/63364
- 235.: WO 00/68374;
- 236.: WO 97/41228;
- 237.: WO 98/45456
- 238.: WO 99/32619
- 239.: WO 99/53050;
- 240.: Xu et al. (1990) Chin J Bot 2(2):81-87
- 241.: Xu ZH et al. (1981) J. Exp. Bot. 32, 767-778.
- 242.: Zhou et al. (1992) Plant Cell Tissue Organ Culture, 30:78-83
- 243.: Zupan et al. (2000) Plant J 23(1):11-28

## Claims

1. A method for producing a transgenic Gramineae plant comprising the steps of:
(a) isolating a zygote from a Gramineae plant to be transformed in a way that said isolated zygote becomes substantially free from its naturally surrounding tissue,
(b) introducing a DNA composition comprising a genetic component into the genome of said Gramineae plant, wherein said introduction is mediated by Agrobacterium transformation into said isolated zygote;
(c) regenerating Gramineae plants from said zygotes which have received said genetic component; and
(d) identifying a fertile, transgenic Gramineae plant whose genome has been altered through the stable introduction of said genetic component.

2. The method of claim 1, wherein the Gramineae plant is selected from the group consisting of wheat, maize and barley.

3. The method of claim 1 or 2, where in the Gramineae plant is a Triticum species.

4. The method of any of claim 1 to 3, wherein the Gramineae plant is regenerated from said isolated zygote by a method comprising co-cultivation of said isolated zygote and/or the zygotic embryo derived therefrom with a feeder system.

5. The method of any of claim 1 to 4, wherein the Gramineae plant is regenerated from said isolated zygote by a method comprising co-cultivation of said isolated zygote and/or the zygotic embryo derived therefrom with a culture of isolated immature pollen or pistils.

6. The method of any of claim 1 to 5, wherein the Gramineae plant is regenerated from said isolated zygote by a method comprising co-cultivation of said isolated zygote and/or the zygotic embryo derived therefrom with
a) a culture of androgenetically developing barley pollen or
b) a culture of wheat or barley pistils or
c) any combination of a) and b).

7. The method of any of claim 1 to 6, wherein the zygotes and the feeder system are physically separated in a way to prevent mixing of the different cell types but to allow exchange of growth factors, proteins, media components, and other low molecular weight compounds.

8. The method of any of claim 1 to 7, wherein co-cultivation of the zygotes and the feeder system are employed already during Agrobacterium co-cultivation in a way that the co-cultivation culture of the zygotes and Agrobacterium is physically separated from the feeder system in a way to prevent contact of the Agrobacteria with the feeder system but to allow exchange of growth factors, proteins, media components, and other low molecular weight compounds.

9. The method of any of claim 1 to 8, wherein said genetic component is transmitted through a complete sexual cycle of said transgenic Gramineae plant to its progeny, wherein said progeny does not comprise a selectable or screenable marker gene.

10. The method of any of claim 1 to 9, wherein said method does not comprise a step which leads to dedifferentiation of the zygote or the zygote-derived embryo.

11. The method of any of claim 1 to 10, wherein said genetic component comprises a expression cassette comprising a nucleic acid sequence operably linked to a promoter active in said Gramineae plant, wherein expression of said nucleic acid sequence confers a phenotypically distinguishable trait to said Gramineae plant.

12. The method of any of claim 1 to 11, wherein the pH of the medium used during co-cultivation of the isolated zygote with Agrobacterium is kept in a range from about 5.8 to about 6.0.
